# EUROPEAN PATENT SPECIFICATION

(11) **EP 1 002 113 B1**
(45) Date of publication and mention of the grant of the patent: **11.03.2009**
(21) Application number: 98926537.6
(22) Date of filing: 11.06.1998
(51) Int. Cl.: C12N 15/82, C12N 9/02, C12N 9/88, C12N 9/12, C12Q 1/68, G01N 33/50, A01H 5/00

(54) **PLANT AMINO ACID BIOSYNTHETIC ENZYMES**
PFLANZLICHE ENZYME DER AMINOSÄUREBIOSYNTHESE
ENZYMES BIOSYNTHETIQUES D'ACIDES AMINES VEGETAUX

(30) Priority: 12.06.1997 US 49406 P; 12.11.1997 US 65385 P
(43) Date of publication of application: 24.05.2000
(73) Proprietor: E.I. DU PONT DE NEMOURS AND COMPANY, Wilmington, DE 19898 (US)
(72) Inventor: FALCO, Saverio, Carl, Wilmington, DE 19810 (US); ALLEN, Stephen, M., West Chester, PA 19382 (US); THORPE, Catherine, Jane, Cambridge CB1 3BU (GB)
(74) Representative: Webber, Philip Michael
(86) International application number: PCT/US1998/012073
(87) International publication number: WO 1998/056935

(56) References cited:
- EP-A- 0 485 970
- WO-A-96/01905
- WO-A-96/38574
- WO-A-97/07665
- US-A- 5 451 516
- US-A- 5 545 545
- PATENT ABSTRACTS OF JAPAN vol. 018, no. 257 (C-1200), 17 May 1994 (1994-05-17) & JP 06 038770 A (MITSUBISHI CORP;OTHERS: 01), 15 February 1994 (1994-02-15)
- NOJI MASAAKI ET AL: "Molecular cloning of a cysteine synthase cDNA from Citrullus vulgaris (watermelon) by genetic complementation in an Escherichia coli Cys- auxotroph" MOLECULAR AND GENERAL GENETICS, vol. 244, no. 1, 1994, pages 57-66, XP002272796 ISSN: 0026-8925
- HELL RUEDIGER ET AL: "Isolation and characterization of two cDNAs encoding for compartment specific isoforms of O-acetylserine (thiol) lyase from Arabidopsis thaliana" FEBS LETTERS, vol. 351, no. 2, 1994, pages 257-262, XP002272797 ISSN: 0014-5793
- YOUSSEFIAN, S., ET AL.: "tobacco plants transformed with the O-acetylserine (thiol) lyase gene of wheat are resistant to toxic levels of hydrogen sulphide gas" THE PLANT JOURNAL, vol. 4, no. 5, 1993, pages 759-769, XP002078206
- SAITO ET AL: "modulation of cysteine biosynthesis in chloroplasts of transgenic tobacco overexpressing cysteine synthase (O-Acetylserine(thiol)-lyase)" PLANT PHYSIOLOGY, AMERICAN SOCIETY OF PLANT PHYSIOLOGISTS, ROCKVILLE, MD, US, no. 106, 1994, pages 887-895, XP002078205 ISSN: 0032-0889
- SAITO, K., ET AL.: "modulation of cysteine biosynthesis in chloroplasts of transgenic tobacco overexpressing cysteine synthase (O-Acetylserine(thiol)-lyase)" PLANT PHYSIOLOGY, vol. 106, 1994, pages 887-895, XP002078205
- YOUSSEFIAN, S., ET AL.: "tobacco plants transformed with the O-acetylserine (thiol) lyase gene of wheat are resistant to toxic levels of hydrogen sulphide gas" THE PLANT JOURNAL, vol. 4, no. 5, 1993, pages 759-769, XP002078206
- BOERJAN, W., ET AL.: "distinct phenotypes generated by overexpression and suppression of S-adenosyl-L-methionine synthetase reveal developmental patterns of gene silencing in tobacco" THE PLANT CELL, vol. 6, October 1994, pages 1401-1414, XP002078207
- RAVANEL, S., ET AL.: "cloning of an arabidopsis thaliana cDNA encoding cystathionine beta-lyase by functional complementation in Escherichia coli" EMBL SEQUENCE DATA LIBRARY,27 March 1995, XP002078265 heidelberg, germany cited in the application

## Description

### FIELD OF THE INVENTION

This invention is in the field of plant molecular biology. More specifically, this invention pertains to nucleic acid fragments encoding enzymes involved in amino acid biosynthesis in plants and seeds.

### BACKGROUND OF THE INVENTION

Many vertebrates, including man, lack the ability to manufacture a number of amino acids and therefore require these amino acids preformed in the diet. These are called essential amino acids. Human food and animal feed, derived from many grains, are deficient in essential Amino acids, such as lysine; the sulfur amino acids methionine and cysteine threonine and tryptophan. For example, in corn (*Zea mays L.*) lysine is the most limiting amino acid for the dietary requirements of many animals. Soybean (*Glycine max L.*) meal is used as an additive to com-based animal feeds primarily as a lysine supplement. Thus an increase in the lysine content of either corn or soybean would reduce or eliminate the need to supplement mixed grain feeds with lysine produced via fermentation of microbes. Furthermore, in corn the sulfur amino acids are the third most limiting amino acids, after lysine and tryptophan, for the dietary requirements of many animals. The use of soybean meal, which is rich in lysine and tryptophan, to supplement corn in animal feed is limited by the low sulfur amino acid content of the legume. Thus, an increase in the sulfur amino acid content of either corn or soybean would improve the nutritional quality of the mixtures and reduce the need for further supplementation through addition of more expensive methionine.

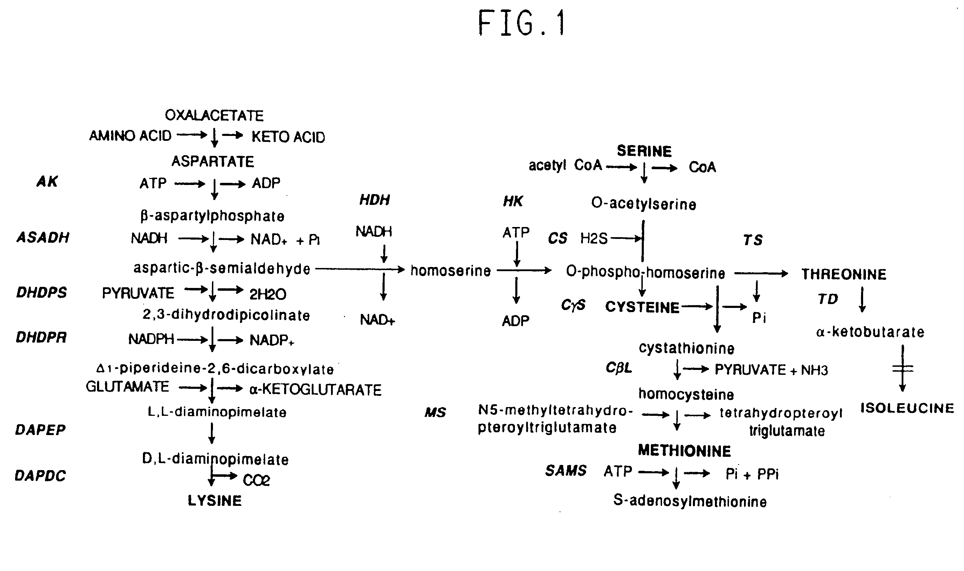

Lysine, threonine, methionine, cysteine and isoleucine are amino acids derived from aspanate. Regulation of the biosynthesis of each member of this family is interconnected (see Figure 1). One approach to increasing the nutritional quality of human foods and animal feed is to increase the production and accumulation of specific free amino acids via genetic engineering of this biosynthetic pathway. Alteration of the activity of enzymes in this pathway could lead to altered levels of lysine, threonine, methionine, cysteine and isoleucine. However, few of the genes encoding enzymes that regulate this pathway in plants, especially corn and soybeans, are available.

The organization of the pathway leading to biosynthesis of lysine; threonine, methionine, cysteine and isoleucine indicates that over-expression or reduction of expression of genes encoding, *inter alia*, aspartic semialedhyde dehydrogenase, homoserine kinase, diaminopimelate decarboxylase, cysteine synthase and cystathionine β-lyase in corn and soybean could be used to alter levels of these amino acids in human food and animal feed. Accordingly, availability of nucleic acid sequences encoding all or a portion of these enzymes would facilitate development of nutritionally improved crop plants.

### SUMMARY OF THE INVENTION

The instant invention relates to isolated nucleic acid fragments encoding plant enzymes involved in amino acid biosynthesis. Specifically, this invention concerns isolated nucleic acid fragments encoding plant cysteine synthase. In addition, this invention relates to nucleic acid fragments that are complementary to nucleic acid fragments encoding the plant cysteine synthase.

In another embodiment, the instant invention relates to chimeric genes encoding the plant cysteine synthase or to chimeric genes that comprise nucleic acid fragments that are complementary to the nucleic acid fragments encoding the plant cysteine synthase operably linked to regulatory sequences which influence the transcription, RNA processing or stability, or translation of the coding sequence, preferably wherein expression of the chimeric genes results in production of levels of the encoded enzymes in transformed host cells that are altered (i.e:, increased or decreased) from the levels produced in untransformed host cells.

In a further embodiment, the instant invention concerns a transformed host cell comprising, preferably in its genome, a chimeric gene encoding a plant cysteine synthase of the invention operably linked to regulatory sequences, which influence the transcription, RNA processing or stability, or translation of the coding sequence. Expression of the chimeric gene results in production of altered levels of the biosynthetic enzyme in the transformed host cell. The transformed host cells can be of eukaryotic or prokaryotic origin, and include cells derived from higher plants and microorganisms.

Also disclosed is a method of altering the level of expression of a plant biosynthetic enzyme in a transformed host cell comprising:
a) transforming a host cell with a chimeric gene comprising a nucleic acid fragment encoding a plant biosynthetic enzyme selected from the group consisting of aspartic semialdehyde dehydrogenase, diaminopimelate decarboxylase, homoserine kinase, cysteine synthase and cystathionine β-lyase, operably linked to suitable regulatory sequences; and
b) growing the transformed host cell under conditions that are suitable for expression of the chimeric gene wherein expression of the chimeric gene results in production of altered levels of the biosynthetic enzyme in the transformed host cell.

Also disclosed is a method for obtaining a nucleic acid fragment encoding all or substantially all of an amino acid sequence encoding a plant aspartic semialedhyde dehydrogenase, diaminopimelate decarboxylase, homoserine kinase, cysteine synthase and cystathionine β-lyase.

Also disclosed is a method for evaluating at least one compound for its ability to inhibit the activity of a plant biosynthetic enzyme selected from the group consisting of aspartic semialedhyde dehydrogenase, diaminopimelate decarboxylase, homoserine kinase, cysteine synthase and cystathionine β-lyase, the method comprising the steps of: (a) transforming a host cell with a chimeric gene comprising a nucleic acid fragment encoding a plant biosynthetic enzyme selected from the group consisting of aspartic semialedhyde dehydrogenase, diaminopimelate decarboxylase, homoserine kinase, cysteine synthase and cystathionine β-lyase, operably linked to suitable regulatory sequences; (b) growing the transformed host cell under conditions that are suitable for expression of the chimeric gene wherein expression of the chimeric gene results in production of the biosynthetic enzyme in the transformed host cell; (c) optionally purifying the biosynthetic enzyme expressed by the transformed host cell; (d) treating the biosynthetic enzyme with a compound to be tested; and (e) comparing the activity of the biosynthetic enzyme that has been treated with a test compound to the activity of an untreated biosynthetic enzyme, thereby selecting compounds with potential for inhibitory activity.

### BRIEF DESCRIPTION OF THE DRAWINGS AND SEQUENCE DESCRIPTIONS

The invention can be more fully understood from the following detailed description and the accompanying drawings and sequence descriptions which form a part of this application.

Figure 1 depicts the biosynthetic pathway for the aspartate family of amino acids. The following abbreviations are used: AK = aspartokinase; ASADH = aspartic semialdehyde dehydrogenase: DHDPS = dihydrodipicolinate synthase; DHDPR = dihydrodipicolinate reductase; DAPEP = diaminopimelate epimerase; DAPDC = diaminopimelate decarboxylase; HDH = homoserine dehydrogenase; HK = homoserine kinase; TS = threonine synthase; TD = threonine deaminase; CγS = cystathionine γ-synthase; CβL = cystathionine β-lyase; MS = methionine synthase; CS = cysteine synthase; and SAMS = S-adenosylmethionine synthase.

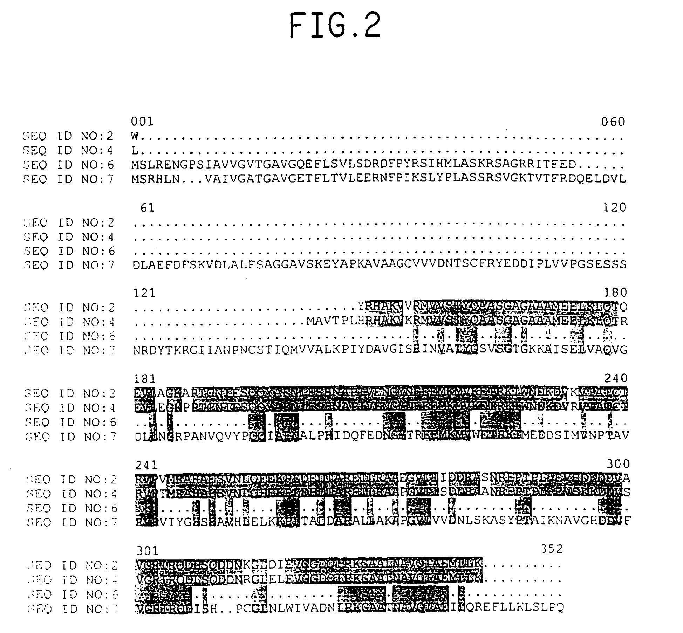

Figures 2 through 6 show the amino acid sequence alignments between the known art sequence for aspartic semialdehyde dehydrogenase, diaminopimelate decarboxylase, homoserine kinase, cysteine synthase, and cystathionine β-lyase vs the sequences included in this application. Alignments were performed using the Clustal alogarithm described in Higgins and Sharp (1989) (CABIOS 5:151-153). Identical amino acids are indicated by gray boxes. In cases where the alignment includes more than two sequences only amino acids which are identical in all sequences are indicated. A description of Figures 2 through 6 follows:
Figure 2 depicts the amino acid sequence alignment between the aspartic semialdehyde dehydrogenases from rice clone rlr.pk003.d11 (SEQ ID NO:2), wheat clone wr1.pk004.c11 (SEQ ID NO:4), soybean clone sf11.pk0122.f9 (SEQ ID NO:6), and *Legionella pneumophila* (GenBank Accession No. AF034213, SEQ ID NO:7).

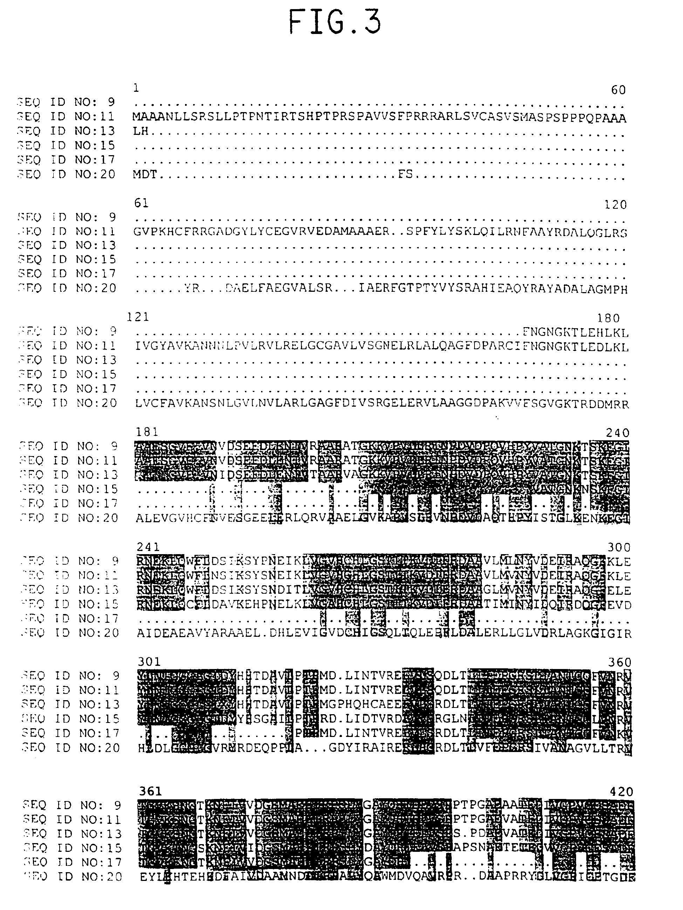

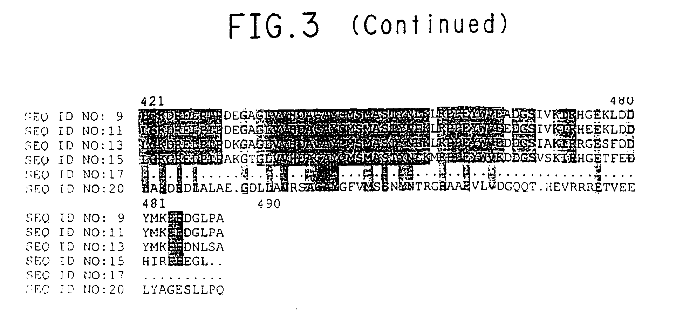

Figure 3 depicts the amino acid sequence alignments between the diaminopimelate decarboxylases from corn clones cen3n.pk0067.a3 (SEQ ID NO:9), crln.pk0103,d8 (SEQ ID NO:11), rice clone r10n.pk0013.b9 (SEQ ID NO: 13), soybean clone sr1.pk0132.c1 (SEQ ID NO:15), wheat clone wlk1.pk0012.c2 (SEQ ID NO:17), and *Pseudomonas aeruginosa* (GenBank Accession No. 118304, SEQ ID NO:20).

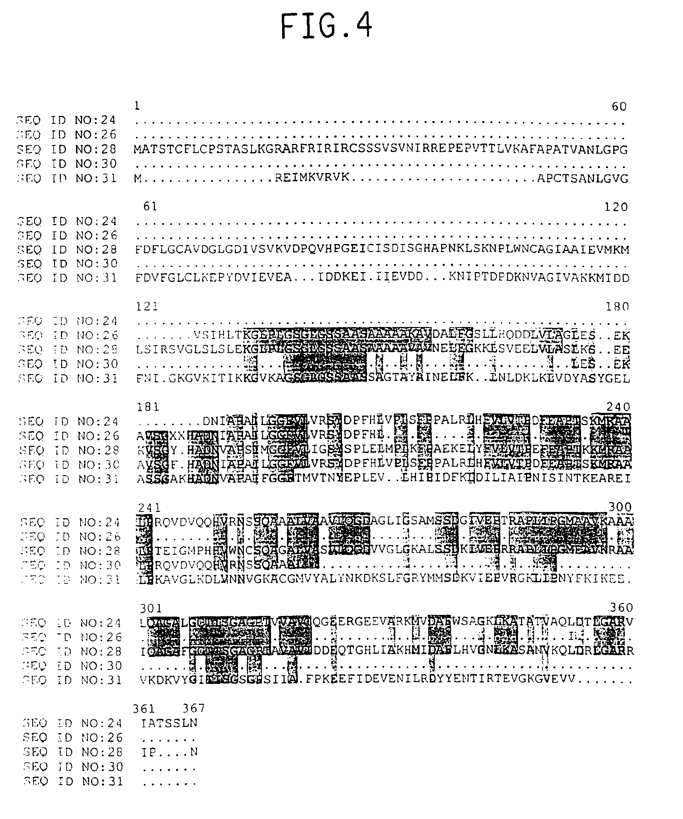

Figure 4 depicts the amino acid sequence alignments between the homoserine kinase from corn clone cr1n.pk0009.g4 (SEQ ID NO:22), rice clone rcalc.pk005.k3 (SEQ ID NO:24), soybean clone ses8w,pk0020.b5 (SEQ ID NO:26), wheat clone wl1n.pk0065.f2 (SEQ ID NO:28), and *Methanococcus jannaschii* (GenBank Accession No. U67553, SEQ ID NO:29).

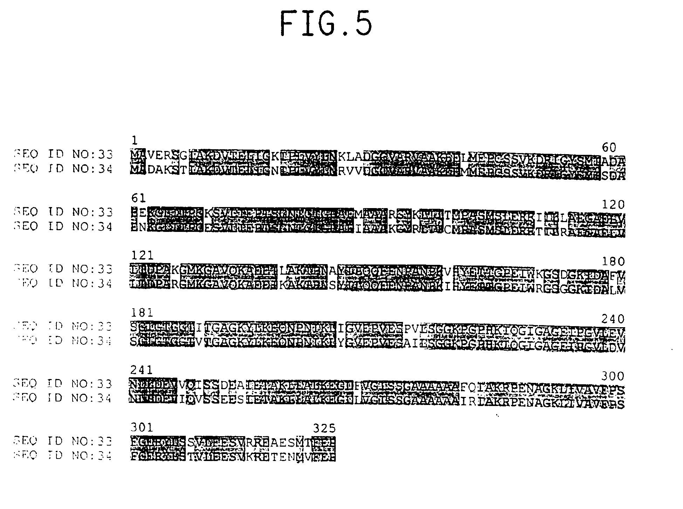

Figure 5 depicts the amino acid sequence alignment between the cysteine synthase from soybean clone se3.05h06 (SEQ ID NO:31), and *Citrullus lanatus (*GenBank Accession No.D28777. SEQ ID NO:32).

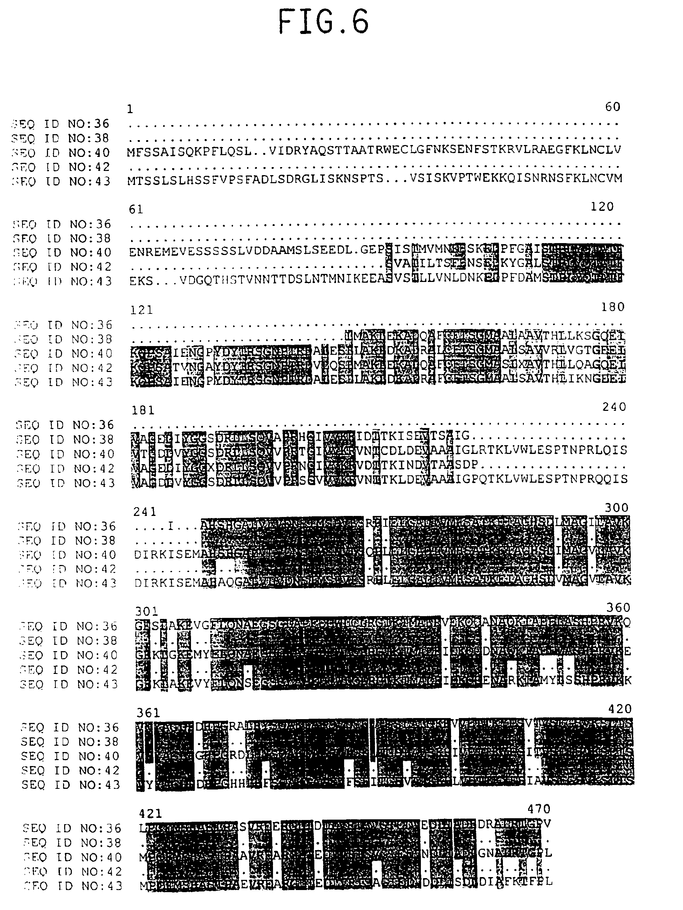

Figure 6 depicts the amino acid sequence alignment between the cystathionine β-lyase from corn clone cen1.pk0061.d4 (SEQ ID NO:34), rice clone rlr12.pk0061.d4 (SEQ ID NO:36), soybean clone sf1l.pk0012.c4 (SEQ ID NO:38), wheat clone wr1.pk0091.g6 (SEQ ID NO:40), and Arabidopsis thaliana (GenBank Accession No. L40511, SEQ ID NO:41).

SEQ ID NO: 1 is the nucleotide sequence comprising the entire cDNA insert in clone rlr48.pk0003.d12 encoding a portion of a rice aspartic-semialdehyde dehydrogenase.
SEQ ID NO:2 is the deduced amino acid sequence of a portion of a rice aspartic-semialdehyde dehydrogenase derived from the nucleotide sequence of SEQ ID NO: 1.
SEQ ID NO:3 is the nucleotide sequence comprising a portion of the cDNA insert in clone wr1.pk0004.c11 encoding a portion of a wheat aspartic-semialdehyde dehydrogenase.
SEQ ID NO:4 is the deduced amino acid sequence of a portion of a wheat aspartic-semialdehyde dehydrogenase derived from the nucleotide sequence of SEQ ID NO:3.
SEQ ID NO:5 is the nucleotide sequence comprising a portion of the cDNA insert in clone sfl1.pk0122.f9 encoding a portion of a soybean aspartic-semialdehyde dehydrogenase.
SEQ ID NO:6 is the deduced amino acid sequence of a portion of a soybean aspartic-semialdehyde dehydrogenase derived from the nucleotide sequence of SEQ ID NO:5.
SEQ ID NO:7 is the amino acid sequence for a *Legionella pneumophila* semialdehyde dehydrogenase found in GenBank Accession No. AF034213.
SEQ ID NO:8 is the nucleotide sequence comprising the entire cDNA insert in clone cen3n.pk0067.a3 encoding a portion of a corn diaminopimelate decarboxylase.
SEQ ID NO:9 is the deduced amino acid sequence of a portion of a corn diaminopimelate decarboxylase derived from the nucleotide sequence of SEQ ID NO:8.
SEQ ID NO:10 is the nucleotide sequence comprising the entire cDNA insert in clone cr1n.pk0103.d8 encoding a portion of a corn diaminopimelate decarboxylase.
SEQ ID NO:11 is the deduced amino acid sequence of a portion of a corn diaminopimelate decarboxylase derived from the nucleotide sequence of SEQ ID NO:10.
SEQ ID NO:12 is the nucleotide sequence comprising the entire cDNA insert in clone r10n.pk0013.b9 encoding a portion of a rice diaminopimelate decarboxylase.
SEQ ID NO:13 is the deduced amino acid sequence of a portion of a rice diaminopimelate decarboxylase derived from the nucleotide sequence of SEQ ID NO:12.
SEQ ID NO:14 is the nucleotide sequence comprising the entire cDNA insert in clone sr1.pk0132.c1 encoding a soybean diaminopimelate decarboxylase.
SEQ ID NO:15 is the deduced amino acid sequence of a portion of a soybean diaminopimelate decarboxylase derived from the nucleotide sequence of SEQ ID NO:14.
SEQ ID NO:16 is the nucleotide sequence comprising a portion of the cDNA insert in clone wlk1.pk0012.c2 encoding a wheat diaminopimelate decarboxylase.
SEQ ID NO:17 is the deduced amino acid sequence of a portion of a wheat diaminopimelate decarboxylase derived from the nucleotide sequence of SEQ ID NO:16.
SEQ ID NO:18 is the nucleotide sequence comprising a portion of the cDNA insert in clone sdp3c.pk001.o15 encoding a soybean diaminopimelate decarboxylase.
SEQ ID NO:19 is the deduced amino acid sequence of a portion of a soybean diaminopimelate decarboxylase derived from the nucleotide sequence of SEQ ID NO:18.
SEQ ID NO:20 is the amino acid sequence of a *Pseudomonas aeruginosa* diaminopimelate decarboxylase found in GenBank Accession No. 118304.
SEQ ID NO:21 is the nucleotide sequence comprising the entire cDNA insert in clone cr1n.pk0009.g4 encoding a corn homoserine kinase.
SEQ ID NO:22 is the deduced amino acid sequence of a portion of a corn homoserine kinase encoded by SEQ ID NO:21.
SEQ ID NO:23 is the nucleotide sequence comprising a portion of the cDNA insert in clone rca1c.pk005.k3 encoding a rice homoserine kinase.
SEQ ID NO:24 is the deduced amino acid sequence of a portion of a rice homoserine kinase encoded by SEQ ID NO:23.
SEQ ID NO:25 is the nucleotide sequence comprising the entire cDNA insert in clone ses8w.pk0020.b5 encoding a soybean homoserine kinase.
SEQ ID NO:26 is the deduced amino acid sequence of a soybean homoserine kinase derived from the nucleotide sequence of SEQ ID NO:25.
SEQ ID NO:27 is the nucleotide sequence comprising a portion of the cDNA insert in clone w11n.pk0065.f2 encoding a wheat homoserine kinase.
SEQ ID NO:28 is the deduced amino acid sequence of a portion of a wheat homoserine kinase derived from the nucleotide sequence of SEQ ID NO:27.
SEQ ID NO:29 is the amino acid sequence of a *Methanococcus jannaschii* homoserine kinase found.in GenBank Accession No. U67553.
SEQ ID NO:30 is the nucleotide sequence comprising the entire cDNA insert in clone se3.05h06 encoding a soybean cysteine synthase.
SEQ ID NO:31 is the deduced amino acid sequence of a soybean cysteine synthase derived from the nucleotide sequence of SEQ ID NO:30.
SEQ ID NO:32 is the amino acid sequence of a *Citrullus lanatus cysteine* synthase found in GenBank Accession No. D28777.
SEQ ID NO:33 is the nucleotide sequence comprising the entire cDNA insert in clone cen1.pk0061.d4 encoding a corn cystathionine β-lyase.
SEQ ID NO:34 is the deduced amino acid sequence of a portion of a corn cystathionine β-lyase derived from the nucleotide sequence of SEQ ID NO:33.
SEQ ID NO:35 is the nucleotide sequence comprising a portion of the cDNA insert in clone r1r12.pk006.g1 encoding a rice cystathionine β-lyase.
SEQ ID NO:36 is the deduced amino acid sequence of a portion of a rice cystathionine β-lyase derived froth the nucleotide sequence of SEQ ID NO:36.
SEQ ID NO:37 is the nucleotide sequence comprising the entire cDNA insert in clone sf11.pk0012.c4 encoding a soybean cystathionine β-lyase.
SEQ ID NO:38 is the deduced amino acid sequence of an entire soybean cystathionine β-lyase derived from the nucleotide sequence of SEQ ID NO:37.
SEQ ID NO:39 is the nucleotide sequence comprising a portion of the cDNA insert in clone wr1.pk0091.g6 encoding a wheat cystathionine β-lyase.
SEQ ID NO:40 is the deduced amino acid sequence of a portion of a wheat cystathionine β-lyase derived from the nucleotide sequence of SEQ ID NO:39.
SEQ ID NO:41 is the amino acid sequence of a *Arabidopsis thaliana* cystathionine β-lyase found in GenBank Accession No. L40511.

The Sequence Descriptions contain the one letter code for nucleotide sequence characters and the three letter codes for amino acids as defined in conformity with the IUPAC-IYUB standards described in *Nucleic Acids Research 13*:3021-3030 (1985) and in the *Biochemical Journal 219 (No. 2)*:345-373 (1984).

### DETAILED DESCRIPTION OF THE INVENTION

In the context of this disclosure, a number of terms shall be utilized. As used herein, an "isolated nucleic acid fragment" is a polymer of RNA or DNA that is single- or double-stranded, optionally containing synthetic, non-natural or altered nucleotide bases. An isolated nucleic acid fragment in the form of a polymer of DNA may be comprised of one or more segments of cDNA, genomic DNA or synthetic DNA.

As used herein, "substantially similar" refers to nucleic acid fragments wherein changes in one or more nucleotide bases results in substitution of one or more amino acids, but do not affect the functional properties of the protein encoded by the DNA sequence. "Substantially similar" also refers to nucleic acid fragments wherein changes in one or more nucleotide bases does not affect the ability of the nucleic acid fragment to mediate alteration of gene expression by antisense or co-suppression technology. "Substantially similar" also refers to modifications of the nucleic acid fragments of the instant invention such as deletion or insertion of one or more nucleotides that do not substantially affect the functional properties of the resulting transcript vis-à-vis the ability to mediate alteration of gene expression by antisense or co-suppression technology or alteration of the functional properties of the resulting protein molecule. It is therefore understood that the invention encompasses more than the specific exemplary sequences.

For example, it is well known in the art that antisense suppression and co-suppression of gene expression may be accomplished using nucleic acid fragments representing less that the entire coding region of a gene, and by nucleic acid fragments that do not share 100% identity with the gene to be suppressed. Moreover, alterations in a gene which result in the production of a chemically equivalent amino acid at a given site, but do not effect the functional properties of the encoded protein, are well known in the art. Thus, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a functionally equivalent product. Nucleotide changes which result in alteration of the N-terminal and C-terminal portions of the protein molecule would also not be expected to alter the activity of the protein. Each of the proposed modifications is well within the routine skill in the art, as is determination of retention of biological activity of the encoded products. Moreover, the skilled artisan recognizes that substantially similar sequences are also defined by their ability to hybridize, under stringent conditions (0.1X SSC, 0.1% SDS, 65°C), with the sequences exemplified herein. Preferred substantially similar nucleic acid fragments of the instant invention are those nucleic acid fragments whose DNA sequences are at least 95% identical to the DNA sequence of the nucleic acid fragments reported herein.

A "substantival portion" of an amino acid or nucleotide sequence comprises enough of the amino acid sequence of a polypeptide or the nucleotide sequence of a gene to afford putative identification of that polypeptide or gene, either by manual evaluation of the sequence by one skilled in the art, or by computer-automated sequence comparison and identification using algorithms such as BLAST (Basic Local Alignment Search Tool; Altschul, S. F., et al., (1993) J. Mol. Biol. 215:403-410; see also www.ncbi.nlm.nih.gov/BLAST/). In general, a sequence of ten or more contiguous amino acids or thirty or more nucleotides is necessary in order to putatively identify a polypeptide or nucleic acid sequence as homologous to a known protein or gene. Moreover, with respect to nucleotide sequences; gene Specific oligonucleotide probes comprising 20-30 continuous nucleotides may be used in sequence-dependent methods of gene identification (e.g., Southern hybridization) and isolation (e.g., *in situ* hybridization of bacterial colonies or bacteriophage plaques). In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to afford specific identification and/or isolation of a nucleic acid fragment comprising the sequence. The instant specification teaches partial or complete amino acid and nucleotide sequences encoding one or more particular plant proteins. The skilled artisan, having the benefit of the sequences as reported herein, may now use all-or a substantial portion of the disclosed sequences for purposes known to those skilled in this art.

"Codon degeneracy" refers to divergence in the genetic code permitting variation of the nucleotide sequence without effecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

"Synthetic genes" can be assembled from oligonucleotide building blocks that are chemically synthesized using procedures known to those skilled in the art. These building blocks are ligated and annealed to form gene segments which are then enzymatically assembled to construct the entire gene. "Chemically synthesized", as related to a sequence of DNA, means that the component nucleotides were assembled *in vitro.* Manual chemical synthesis of DNA may be accomplished using well established procedures, or automated chemical synthesis can be performed using one of a number of commercially available machines. Accordingly, the genes can be tailored for optimal gene expression based on optimization of nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell where sequence information is available.

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

"Coding sequence" refers to a DNA sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, and polyadenylation recognition sequences.

"Promoters" refers to a DNA sequence capable of controlling the expression of a coding sequence or functional RNA. In general, a coding sequence is located 3' to a promoter sequence. The promoter sequence consists of proximal and more distal upstream elements, the latter elements often referred to as enhancers. Accordingly, an "enhance" is a DNA sequence which can stimulate promoter activity and may be an innate element of the promoter or a heterologous element inserted to enhance the level or tissue-specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. Promoters which cause a gene to be expressed in most cell types at most times are commonly referred to as "constitutive promoters". New promoters of various types useful in plant cells are constantly being discovered; numerous examples may be found in the compilation by Okamuro and Goldberg, (1989) Biochemistry of Plants 15:1-82. It is further recognized that since in most cases the exact boundaries of regulatory sequences have not been completely defined, DNA fragments of different lengths may have identical promoter activity.

The "translation leader sequence" refers to a DNA sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner, R. and Foster, G.D. (1995) Molecular Biotechnology 3:225).

The "3' non-coding sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) Plant Cell 1:671-680.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from posttranscriptional processing of the primary transcript and is referred to as the mature RNA. "Messenger RNA (mRNA)" refers to the RNA that is without introns and that can be translated into protein by the cell. "cDNA" refers to a double-stranded DNA that is complementary to and derived from mRNA. "Sense" RNA refers to RNA transcript that includes the mRNA and so can be translated into protein by the cell. "Antisense RNA" refers to a RNA transcript that is complementary to all or part of a target primary transcript or mRNA and that blocks the expression of a target gene (U.S. Pat. No. 5,107,065). The complementarity of an antisense RNA may be with any part of the specific gene transcript, i.e., at the 5' non-coding sequence, 3' non-coding sequence, introns, or the coding sequence. "functional RNA" refers to antisense RNA, ribozyme RNA, or other RNA that is not translated yet has an effect on cellular processes.

The term "operably linked" refers to the association of nucleic acid sequences on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

The term "expression", as used herein, refers to the transcription and stable accumulation of sense (mRNA) or antisense RNA derived from the nucleic acid fragment of the invention. Expression may also refer to translation of mRNA into a polypeptide. "Antisense inhibition" refers to the production of antisense RNA transcripts capable of suppressing the expression of the target protein. "Overexpression" refers to the production of a gene product in transgenic organisms that exceeds levels of production in normal or non-transformed organisms. "Co-suppression" refers to the production of sense RNA transcripts capable of suppressing the expression of identical or substantially similar foreign or endogenous genes (U.S. Pat. No. 5,231,020).

"Altered levels" refers to the production of gene product(s) in transgenic organisms in amounts or proportions that differ from that of normal or non-transformed organisms.

"Mature" protein refers to a post-translationally processed polypeptide; i.e., one from which any pre- or propeptides present in the primary translation product have been removed. "precursor" protein refers to the primary product of translation of mRNA; i.e.. with pre- and propeptides still present. Pre- and propeptides may be but are not limited to intracellular localization signals.

A "chloroplast transit peptide" is an amino acid sequence which is translated in conjunction with a protein and directs the protein to the chloroplast or other plastid types present in the cell in which the protein is made. "Chloroplast transit sequence" refers to a nucleotide sequence that encodes a chloroplast transit peptide. A "signal peptide" is an amino acid sequence which is translated in conjunction with a protein and directs the protein to the secretory system (Chrispeels, J.J., (1991) Ann. Rev. Plant Phys. Plant Mol. Biol. 42:21-53). If the protein is to be directed to a vacuole, a vacuolar targeting signal (*supra*) can further be added, or if to the endoplasmic reticulum, an endoplasmic reticulum retention signal (*supra*) may be added. If the protein is to be directed to the nucleus, any signal peptide present should be removed and instead a nuclear localization signal included (Raikhel (1992) Plant Phys. 100:1627-1632).

"Transformation" refers to the transfer of a nucleic acid fragment into the genome of a host organism, resulting in genetically stable inheritance. Host organisms containing the transformed nucleic acid fragments are referred to as "transgenic" organisms. Examples of methods of plant transformation include Agrobacterium-mediated transformation (De Blaere et al. (1987) Meth. Enzymol. 143:277) and particle-accelerated or "gene gun" transformation technology (Klein et al. (1987) Nature (London) 327:70-73; U.S. Pat. No. 4,945,050).

Standard recombinant DNA and molecular cloning techniques used herein are well known in the art and are described more fully in Sambrook, J., Fritsch, E.F. and Maniatis, T. Molecular Cloning: A Laboratory Manual; Cold Spring Harbor Laboratory Press: Cold Spring Harbor, 1989 (hereinafter "Maniatis").

Nucleic acid fragments encoding at least a portion of several plant amino acid biosynthetic enzymes have been isolated and identified by comparison of random plant cDNA sequences to public databases containing nucleotide and protein sequences using the BLAST algorithms well known to those skilled in the art. Table 1 lists the amino acid biosynthetic enzymes that are described herein, and the designation of the cDNA clones that comprise the nucleic acid fragments encoding these enzymes.

**TABLE 1**

| Amino Acid Biosynthetic Enzymes | | |
|---|---|---|
| Enzyme | Clone | Plant |
| aspartic semialedhyde dehydrogenase | rlr48.pk003.d12 | rice |
| | wr1.pk0004.c11 | wheat |
| | sf11.pk0122.f9 | soybean |
| diaminopimelate decarboxylase | cen3n.pk0067.a3 | corn |
| | cr1n.pk0103.d8 | corn |
| | r10n.pk0013.b9 | rice |
| | sr1.pk0132.c1 | soybean |
| | wlk1.pk0012.c2 | wheat |
| | sdp3c.pk001.o15 | soybean |
| homoserine kinase | crln.pk0009.g4 | corn |
| | rcalc.pk005.k3 | rice |
| | ses8w.pk0020.b5 | soybean |
| | w11n.pk0065.f2 | wheat |
| cysteine synthase | se3.05h06 | soybean |
| cystathionine β-lyase | cen1.pk0061.d4 | corn |
| | rlr12.pk0026.g1 | rice |
| | sf11.pk0012.c4 | soybean |
| | wr1.pk0091.g6 | wheat |

The nucleic acid fragments of the instant invention may be used to isolate cDNAs and genes encoding homologous enzymes from the same or other plant species. Isolation of homologous genes using sequence-dependent protocols is well known in the art. Examples of sequence-dependent protocols include, but are not limited to, methods of nucleic acid hybridization, and methods of DNA and RNA amplification as exemplified by various uses of nucleic acid amplification technologies (e.g.. polymerase chain reaction, ligase chain reaction).

For example, genes encoding other amino acid biosynthetic enzymes, either as cDNAs or genomic DNAs, could be isolated directly by using all or a portion of the instant nucleic acid fragments as DNA hybridization probes to screen libraries from any desired plant employing methodology well known to those skilled in the art. Specific oligonucleotide probes based upon the instant nucleic acid sequences can be designed and synthesized by methods known in the art (Maniatis). Moreover, the entire sequences can be used directly to synthesize DNA probes by methods known to the skilled artisan such as random primers DNA labeling, nick translation, or end-labeling techniques, or RNA probes using available *in vitro* transcription systems. In addition, specific primers can be designed and used to amplify a part of or full-length of the instant sequences. The resulting amplification products can be labeled directly during amplification reactions or labeled after amplification reactions, and used as probes to isolate full length cDNA or genomic fragments under conditions of appropriate stringency.

In addition, two short segments of the instant nucleic acid fragments may be used in polymerase chain reaction protocols to amplify longer nucleic acid fragments encoding homologous genes from DNA or RNA. The polymerase chain reaction may also be performed on a library of cloned nucleic acid fragments wherein the sequence of one primer is derived from the instant nucleic acid fragments, and the sequence of the other primer takes advantage of the presence of the polyadenylic acid tracts to the 3' end of the mRNA precursor encoding plant genes. Alternatively, the second primer sequence may be based upon sequences derived from the cloning vector. For example, the skilled artisan can follow the RACE protocol (Frohman et al., (1988) Proc. Natl. Asoc. Sci. USA 85:8998) to generate cDNAs by using PCR to amplify copies of the region between a single point in the transcript and the 3' or 5' end. Primers oriented in the 3' and 5' directions can be designed from the instant sequences. Using commercially available 3' RACE or 5' RACE systems (BRL), specific 3' or 5' cDNA fragments can be isolated (Ohara et al., (1989) Proc. Natl. Asoc. Sci. USA 86:5673; Loh et al., (1989) Science 243:217). Products generated by the 3' and 5' RACE procedures can be combined to generate full-length cDNAs (Frohman. M.A. and Martin, G.R., (1989) Techniques 1:165).

Availability of the instant nucleotide and deduced amino acid sequences facilitates immunological screening cDNA expression libraries. Synthetic peptides representing portions of the instant amino acid sequences may be synthesized. These peptides can be used to immunize animals to produce polyclonal or monoclonal antibodies with specificity for peptides or proteins comprising the amino acid sequences. These antibodies can be then be used to screen cDNA expression libraries to isolate full-length cDNA clones of interest (Lerner, R.A. (1984) Adv. Immunol. 36:1; Maniatis).

The nucleic acid fragments of the instant invention may be used to create transgenic plants in which the disclosed biosynthetic enzymes are present at higher or lower levels than normal or in cell types or developmental stages in which they are not normally found. This would have the effect of altering the level of free amino acids in those cells.

Overexpression of the biosynthetic enzymes of the instant invention may be accomplished by first constructing chimeric genes in which the coding region are operably linked to promoters capable of directing expression of a gene in the desired tissues at the desired stage of development. For reasons of convenience, the chimeric genes may comprise promoter sequences and translation leader sequences derived from the same genes. 3' Non-coding sequences encoding transcription termination signals may also be provided. The instant chimeric genes may also comprise one or more introns in order to facilitate gene expression.

Plasmid vectors comprising the instant chimeric genes can then constructed. The choice of plasmid vector is dependent upon the method that will be used to transform host plants. The skilled artisan is well aware of the genetic elements that must be present on the plasmid vector in order to successfully transform, select and propagate host cells containing the chimeric gene. The skilled artisan will also recognize that different independent transformation events will result in different levels and patterns of expression (Jones et al., (1985) EMBO J. 4:2411-2418; De Almeida et al., (1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, Western analysis of protein expression, or phenotypic analysis.

For some applications it may be useful to direct the instant biosynthetic enzyme to different cellular compartments, or to facilitate its secretion from the cell. It is thus envisioned that the chimeric genes described above may be further supplemented by altering the coding sequences to encode enzymes with appropriate intracellular targeting sequences such as transit sequences (Keegstra, K. (1989) Cell 56:247-253), signal sequences or sequences encoding endoplasmic reticulum localization (Chrispeels. J.J., (1991) Ann. Rev. Plant Phys. Plant Mol. Biol. 42:21-53), or nuclear localization signals (Raikhel, N. (1992) Plant Phys.100:1627-1632) added and/or with targeting sequences that are already present removed. While the references cited give examples of each of these, the list is not exhaustive and more targeting signals of utility may be discovered in the future.

It may also be desirable to reduce or eliminate expression of the genes encoding the instant biosynthetic enzymes in plants for some applications. In order to accomplish this, chimeric genes designed for co-suppression of the instant biosynthetic enzymes can be constructed by linking the genes or gene fragments encoding the enzymes to plant promoter sequences. Alternatively, chimeric genes designed to express antisense RNA for all or part of the instant nucleic acid fragments can be constructed by linking the genes or gene fragment in reverse orientation to plant promoter sequences. Either the co-suppression or antisense chimeric genes could be introduced into plants via transformation wherein expression of the corresponding endogenous genes are reduced or eliminated.

The instant amino acid biosynthetic enzymes (or portions of the enzymes) may be produced in heterologous host cells, particularly in the cells of microbial hosts, and can be used to prepare antibodies to the enzymes by methods well known to those skilled in the art. The antibodies are useful for detecting the enzymes *in situ* in cells or *in vitro* in cell extracts. Preferred heterologous host cells for production of the instant amino acid biosynthetic enzymes are microbial hosts. Microbial expression systems and expression vectors containing regulatory sequences that direct high level expression of foreign proteins are well known to those skilled in the art. Any of these could be used to construct chimeric genes for production of the instant amino acid biosynthetic enzymes. These chimeric genes could then be introduced into appropriate microorganisms via transformation to provide high level expression of the enzymes. An example of a vector for high level expression of the instant amino acid biosynthetic enzymes in a bacterial host is provided (Example 6).

Additionally, the instant plant amino acid biosynthetic enzymes can be used as a targets to facilitate design and/or identification of inhibitors of the enzymes that may be useful as herbicides. This is desirable because the enzymes described herein catalyze various steps in a pathway leading to production of several essential amino acids. Accordingly, inhibition of the activity of one or more of the enzymes described herein could lead to inhibition of amino acid biosynthesis sufficient to inhibit plant growth. Thus, the instant plant amino acid biosynthetic enzymes could be appropriate for new herbicide discovery and design.

All or a substantial portion of the nucleic acid fragments of the instant invention may also be used as probes for genetically and physically mapping the genes that they are a part of, and as markers for traits linked to those genes. Such information may be useful in plant breeding in order to develop lines with desired phenotypes. For example, the instant nucleic acid fragments may be used as restriction fragment length polymorphism (RFLP) markers. Southern blots (Maniatis) of restriction-digested plant genomic DNA may be probed with the nucleic acid fragments of the instant invention. The resulting banding patterns may then be subjected to genetic analyses using computer programs such as MapMaker (Lander et at., (1987) Genomics 1:174-181) in order to construct a genetic map. In addition, the nucleic acid fragments of the instant invention may be used to probe Southern blots containing restriction endonuclease-treated genomic DNAs of a set of individuals representing parent and progeny of a defined genetic cross. Segregation of the DNA polymorphisms is noted and used to calculate the position of the instant nucleic acid sequence in the genetic map previously obtained using this population (Botstein, D. et al., (1980) Am. J. Hum. Genet. 32:314-331).

The production and use of plant gene-derived probes for use in genetic mapping is described in R. Bernatzky, R. and Tanksley, S. D. (1986) Plant Mol. Biol. Reporter 4(1):37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

Nucleic acid probes derived from the instant nucleic acid sequences may also be used for physical mapping (i.e., placement of sequences on physical maps; *see* Hoheisel, J. D., et al., In: Nonmammalian Genomic Analysis: A Practical Guide, Academic press 1996, pp. 319-346, and references cited therein).

In another embodiment, nucleic acid probes derived from the instant nucleic acid sequences may be used in direct fluorescence *in situ* hybridization (FISH) mapping (Trask, B. J. (1991) Trends Genet. 7:149-154). Although current methods of FISH mapping favor use of large clones (several to several hundred KB; see Laan, M. et al. (1995) Genome Research 5:13-20), improvements in sensitivity may allow performance of FISH mapping using shorter probes.

A variety of nucleic acid amplification-based methods of genetic and physical mapping may be carried out using the instant nucleic acid sequences. Examples include allele-specific amplification (Kazazian, H. H. (1989) J. Lab. Clin. Med. 114(2):95-96), polymorphism of PCR-amplified fragments (CAPS; Sheffield, V. C. et al. (1993) Genomics 16:325-332), allele-specific ligation (Landegren, U. et al. (1988) Science 241:1077-1080), nucleotide extension reactions (Sokolov, B. P. (1990) Nucleic Acid Res. 18:3671), Radiation Hybrid Mapping (Walter, M. A. et al. (1997) Nature Genetics 7:22-28) and Happy Mapping (Dear, P. H. and Cook, P. R. (1989) Nucleic Acid Res. 17:6795-6807). For these methods, the sequence of a nucleic acid fragment is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Loss of function mutant phenotypes may be identified for the instant cDNA clones either by targeted gene disruption protocols or by identifying specific mutants for these genes contained in a maize population carrying mutations in all possible genes (Ballinger and Benzer, (1989) Proc. Natl. Acad. Sci USA 86:9402; Koes et al., (1995) Proc. Natl. Acad. Sci USA 92:8149; Bensen et al., (1995) Plant Cell 7:75). The latter approach may be accomplished in two ways. First, short segments of the instant nucleic acid fragments may be used in polymerase chain reaction protocols in conjunction with a mutation tag sequence primer on DNAs prepared from a population of plants in which Mutator transposons or some other mutation-causing DNA element has been introduced (see Bensen. *supra*). The amplification of a specific DNA fragment with these primers indicates the insertion of the mutation tag element in or near the plant gene encoding the instant amino acid biosynthetic enzymes. Alternatively, the instant nucleic acid fragment may be used as a hybridization probe against PCR amplification products generated from the mutation population using the mutation tag sequence primer in conjunction with an arbitrary genomic site primer, such as that for a restriction enzyme site-anchored synthetic adaptor. With either method, a plant containing a mutation in the endogenous gene encoding an aspartic semialedhyde dehydrogenase, diaminopimelate decarboxylase, homoserine kinase, cysteine synthase or cystathionine β-lyase can be identified and obtained. This mutant plant can then be used to determine or confirm the natural function of the aspartic semialedhyde dehydrogenase, diaminopimelate decarboxylase, homoserine kinase, cysteine synthase or cystathionine β-lyase gene product.

### EXAMPLES

The present invention is further defined in the following Examples, in which all parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions.

### EXAMPLE 1

### Composition of cDNA Libraries; Isolation and Sequencing of cDNA Clones cDNA libraries representing mRNAs from various corn, rice, soybean and wheat tissues were prepared. The characteristics of the libraries are described below.

**TABLE 2**

| cDNA Libraries from Corn, Soybean and Other Plant Tissues | | |
|---|---|---|
| Library | Tissue | Clone |
| cen1 | Corn Endosperm 12 Days After Pollination | cen1.pk0061.d4 |
| cen3n | Corn Endosperm 20 Days After Pollination* | cen3n.pk0067.a3 |
| cr1n | Corn Root From 7 Day Seedlings* | cr1n.pk0009.g4 |
| | | cr1n.pk0103.d8 |
| rca1c | Rice Nipponbare callus | rca1c.pk005.k3 |
| r10n | Rice Leaf 15 Days After Germination* | r10n.pk0013.b9 |
| r1r12 | Rice Leaf 15 Days After Germination. 12 hours after | r1r12.pk0026.g1 |
| | infection of strain *Magaporthe grisea* 4360-R-62 (AVR2- | |
| | YAMO) | |
| r1r48 | Rice Leaf 15 Days After. Germination 48 hours after | r1r48.pk003.d12 |
| | infection of strain *Magaporthe grisea* 4360-R-62 (AVR2- | |
| | YAMO) | |
| sdp3c | Soybean Developing Pods 8-9 mm | sdp3c.pk001.o15 |
| se3 | Soybean Embryo 13 Days After Flowering | se3.05h06 |
| ses8w | Mature Soybean Embryo 8 Weeks After Subculture | ses8w.pk0020.b5. |
| sf11 | Soybean Immature Flower | sf11.pk0012.c4 |
| sr1 | Soybean Root From 10 Day Old Seedlings | sr1.pk0132.c1 |
| w11n | Wheat Leaf from 7 Day Old Seedling* | w11n.pk0065.f2 |
| w1k1 | Wheat Seedlings 1 hour After Fungicide Treatment** | w1k1.pk0012.c2 |
| wr1 | Wheat Root From 7 Day Old Seedlings | wr1.pk0004.c11 |
| | | wr1.pk0091.g6 |

| | | |
|---|---|---|
| *These libraries were normalized essentially as described in U.S. Pat. No. 5,482,845 **Application of 6-iodo-2-propoxy-3-propyl-4(3*H*)-quinazolinone; synthesis and methods of using this compound are described in USSN 08/545,827, | | |

cDNA libraries were prepared in Uni-ZAP™ XR vectors according to the manufacturer's protocol (Stratagene Cloning Systems, La Jolla, CA). Conversion of the Uni-ZAP™ XR libraries into plasmid libraries was accomplished according to the protocol provided by Stratagene. Upon conversion, cDNA inserts were contained in the plasmid vector pBluescript. cDNA inserts from randomly picked bacterial colonies containing recombinant pBluescript plasmids were amplified via polymerase chain reaction using primers specific for vector sequences flanking the inserted cDNA sequences or plasmid DNA was prepared from cultured bacterial cells. Amplified insert DNAs or plasmid DNAs were sequenced in dye-primer sequencing reactions to generate partial cDNA sequences (expressed sequence tags or "ESTs"; see Adams, M. D. et al., (1991) Science 252:1651). The resulting ESTs were analyzed using a Perkin Elmer Model 377 fluorescent sequencer.

### EXAMPLE 2

### Identification and Characterization of cDNA Clones

ESTs encoding plant amino acid biosynthetic enzymes were identified by conducting BLAST (Basic Local Alignment Search Tool; Altschul, S. F., et al., (1993) J. Mol. Biol. 215:403-410; see also www.ncbi.nlm.nih.gov/BLAST/) searches for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The cDNA sequences obtained in Example 1 were analyzed for similarity to all publicly available DNA sequences contained in the "nr" database using the BLASTN algorithm provided by the National Center for Biotechnology Information (NCBI). The DNA sequences were translated in all reading frames and compared for similarity to all publicly available protein sequences contained in the "nr" database using the BLASTX algorithm (Gish. W. and States, D. J. (1993) Nature Genetics 3:266-272) provided by the NCBI. For convenience, the P-value (probability) of observing a match of a cDNA sequence to a sequence contained in the searched databases merely by chance as calculated by BLAST are reported herein as "pLog" values, which represent the negative of the logarithm of the reported P-value. Accordingly, the greater the pLog value, the greater the likelihood that the cDNA sequence and the BLAST "hit" represent homologous proteins.

The BLASTX search using the nucleotide sequences from clones rlr48.pk0003.d12 and wr1.pk0004.c11 revealed similarity of the protein encoded by the cDNAs to *Synechocystis sp*. aspartate semialdehyde dehydrogenase (DDJB Accession No. D64006; r1r48.pk0003.d12 pLog = 44.00; wr1.pk0004.c11 pLog = 34.89). The BLASTX search using the entire cDNA inserts in clones rlr48.pk0003.d12 and wrl.pk0004.c11 revealed a higher pLog value vs. the *Synechocystis* sp protein and similarity of the protein encoded by the cDNAs to *Legionella pneumophila* aspartate semialdehyde dehydrogenase (GenBank Accession No. AF034213). The BLASTX search using the nucleotide sequence from clone sf11.pk0122.f9 revealed similarity of the protein encoded by the cDNA to *Legionella pneumophila*. The BLAST results for these sequences is shown in Table 3:

**TABLE 3**

| BLAST Results for Clones Encoding Polypeptides Homologous to Aspartate Semialdehyde Dehydrogenase | | |
|---|---|---|
| | BLAST pLog Score | |
| Clone | D64006 (*Synechocystis* sp) | AF034213 *(L. pneumophila)* |
| rlr48.pk0003.d12 | 51.00 | 36.00 |
| wr1.pk0004.c11 | 67.96 | 44.74 |
| sf11.pk0122.f9 | | 6.60 |

The sequence of the entire cDNA insert in clone rlr48.pk0003.d12 is set forth in SEQ ID NO:1; the deduced amino acid sequence is set forth in SEQ ID NO:2. The sequence of the entire cDNA insert in clone wrl.pk0004.c11 is set forth in SEQ ID NO:3: the deduced amino acid sequence is set forth in SEQ ID NO:4. The sequence of a portion of the cDNA insert from clone sf11.pk0122.f9 is set forth in SEQ ID NO:5; the deduced amino acid sequence is set forth in SEQ ID NO:6. Sequence alignments and BLAST scores and probabilities indicate that the instant nucleic acid fragments encode a portion of a rice (r1r48.pk0003.d12), wheat (wrl.pk0004.c11), and soybean (sf11.pk0122.f9) aspartate semialdehyde dehydrogenase enzyme. These sequences represent the first plant sequences identified for aspartate semialdehyde dehydrogenase. As depicted in Figure 1, the rice and wheat sequences are 94% identical over 204 amino acids and 58% identical to the *Legionella pneumophila* sequence. The soybean sequence aligns to a more 5' region of the *Legionella pneumophila* sequence with 44% identity over 54 amino acids.

The BLASTX search using the nucleotide sequences from clones cen3n.pk0067.a3, cr1n.pk0103.d8. r10n.pk0013.b9, sr1.pk0132.c1, and wlk1.pk0012.c2 revealed similarity of the proteins encoded by the cDNAs to *Aquifex aeolicus* (GenBank Accession No. AE000728) and *Pseudomonas aeruginosa* (GenBank Accession No. M23174) diaminopimelate decarboxylase. The BLAST results for each of these ESTs are shown in Table 4:

**TABLE 4**

| BLAST Results for Clones Encoding Polypeptides Homologous to Diaminopimelate Decarboxylase | | |
|---|---|---|
| | BLAST pLog Score | |
| Clone | AE000728 (*A. aeolicus)* | M23174 (*P. aeruginosa)* |
| cen3n.pk0067.a3 | 58.22 | 56.00 |
| cr1n.pk0103.d8 | 75.25 | 79.12 |
| r10n.pk0013 | 46.40 | 44.00 |
| sr1.pk0132.c1 | 44.70 | 39.15 |
| w1k1.pk0012.c2 | 20.48 | 19.05 |

The nucleotide sequence of the entire cDNA insert in clone cen3n.pk0067.a3 was determined and is set forth in SEQ ID NO:8, the deduced amino acid sequence of this cDNA is shown in SEQ ID NO:9. The nucleotide sequence of the entire cDNA insert in clone cr1n.pk0103.d8 was determined and is set forth in SEQ ID NO:10. the deduced amino acid sequence of this cDNA is shown in SEQ ID NO:11. The nucleotide sequence of the entire cDNA insert in clone r10n.pk0013.b9 was determined and is set forth in SEQ ID NO:12, the deduced amino acid sequence of this cDNA is shown in SEQ ID NO:13. The sequence of the entire cDNA insert in clone sr1.pk0132.c1 was determined and is set forth in SEQ ID NO:14; the deduced amino acid sequence of this cDNA is shown in SEQ ID NO:15. The sequence of a portion of the cDNA insert from clone wlk1.pk0012.c2 is set forth in SEQ ID NO:16, the deduced amino acid sequence of this cDNA is shown in SEQ ID NO:17. The deduced amino acid sequence of cDNA clone sr1.pk0132.c1 was then used to query the cDNA sequences obtained in Example 1 using the TFASTA algorithm (Pearson, W. R. (1990) Methods in Enzymology 183:63-98). An additional cDNA clone. sdpc.pk001.o15. was identified as sharing homology with sr1.pk0132.c1. BLASTX search using the nucleotide sequences from clone sdpc.pk001.o15 revealed similarity of the proteins encoded by the cDNA to *Pseudomonas fluorescens* diaminopimelate decarboxylase (EMBO Accession No. Y 12268; pLog = 8.66). The sequence of a portion of the cDNA insert in clone sdpc.pk001.o15 is set forth in SEQ ID NO:18; the deduced amino acid sequence of this cDNA is shown in SEQ ID NO:19. The deduced amino acid sequences show significant sequence identity to the *Aquifex aeolicus* and *Pseudomonas aeruginosa* diaminopimelate decarboxylase enzymes. Sequence alignments and BLAST scores and probabilities indicate that the instant nucleic acid fragments encode at least a portion of corn (cen3n.pk0067.a3 and cr1n.pk0103.d8), rice (r10n.pk0013.b9), soybean (sr1.pk0132.c1 and sdpc.pk001.o15), and wheat (w1k1.pk0012.c2) diaminopimelate decarboxylase enzymes. These are the first plant sequences identified that encode a diaminopimelate decarboxylase enzyme. The alignment shown in Figure 3 indicates that the corn sequences (cen3n.pk0067.a3 and cr1n.pk0103.d8) are 98% identical over 323 amino acids. The full-length corn sequence (cr1n.pk0103.d8) is 90.52% identical to the rice sequence (r10n.pk001.3.b9) over 306 amino acids, 96% identical to the wheat sequence (w1k1.pk0012.c2) over 73 amino acids, and 78.46% identical to the soybean full insert sequence (sr1.pk0132.c1) over 260 amino acids. The same corn sequence is 35.29% identical to the *Pseudomonas aeruginosa* diaminopimelate decarboxylase enzyme.

The BLASTX search using the nucleotide sequences from clones cr1n.pk0009.g4, rca1c.pk005.k3, ses8w.pk0020.b5, and w11n.pk0065.f2 revealed similarity of the protein encoded by the cDNA to *Methanococcus jannaschii* homoserine kinase enzyme (GenBank Accession No. U67553). The BLAST results for each of these sequences are shown in Table 5:

**TABLE 5**

| BLAST Results for Clones Encoding Polypeptides Homologous to Homoserine Kinase | |
|---|---|
| | BLAST pLog Score |
| Clone | GenBank Accession No U67553 |
| cr1n.pk0009.g4 | 19.30 |
| rca1c.pk005.k3 | 15.21 |
| ses8w.pk0020.b5 | 35.30 |
| w11n.pk0065.f2 | 5.68 |

The nucleotide sequence of the entire cDNA insert in clone cr1n.pk0009.g4 was determined and is set forth in SEQ ID NO:21; the amino acid sequence deduced from this nucleotide sequence is set forth in SEQ ID NO:22. The sequence of a portion of the cDNA insert from clone rca1c.pk005.k3 is set forth in SEQ ID NO:23, the deduced amino acid sequence of this cDNA is shown in SEQ ID NO:24. The sequence of the entire cDNA insert in clone ses8w.pk0020.b5 was determined and is set forth in SEQ ID NO:25; the deduced amino acid sequence of this cDNA is shown in SEQ ID NO:26. The sequence of a portion of the cDNA insert from clone w11n.pk0065.f2 is set forth in SEQ ID NO:27, the deduced amino acid sequence of this cDNA is shown in SEQ ID NO:28. Sequence alignments and BLAST scores and probabilities indicate that the instant nucleic acid fragments encode portions of a corn (cr1n.pk0009.g4), rice (rca1c.pk005.k3), soybean (ses8w.pk0020.b5), and wheat (w11n.pk0065f2) homoserine kinase enzyme. These sequences represent the first plant sequences encoding homoserine kinase. An alignment of the deduced amino acid sequences with the sequence of the *Methanococcus jannaschii* homoserine kinase enzyme shown in Figure 4 indicates that the corn (cr1n.pk0009.g4) sequence is 100% identical to the rice sequence (rca1c.pk005.k3) over 22 amino acids. The corn sequence is 100% identical to the wheat sequence (w11n.pk0065f2) over 72 amino acids. The corn sequence is 60.35% identical to the soybean sequence (ses8w.pk0020.b5) over 179 amino acids. The soybean clone (ses8w.pk0020.b5) contains the sequence for a full-length homoserine kinase and is 34.67% identical to the *Methanococcus jannaschii* sequence.

The BLASTX search using the nucleotide sequence of clone se3.05h06 revealed similarity of the protein encoded by the cDNA to *Citrullus lanatus* cysteine synthase (DDJB Accession No. D28777; pLog = 59.06). The sequence of the entire cDNA insert in clone se3.05h06 was determined and is set forth in SEQ ID NO:30; the deduced amino acid sequence of this cDNA is shown in SEQ ID NO:31. The entire cDNA insert in clone se3.05h06 was reevaluated by BLAST, yielding an even higher pLog value vs. the *Citrullus lanatus* cysteine synthase (D28777; pLog = 182.64). Sequence alignments and BLAST scores and probabilities indicate that the instant nucleic acid fragment encodes the entire soybean cysteine synthase enzyme. This is the first soybean EST identified for cysteine synthase. Figure 5 shows the alignment of the amino acid sequence deduced from the clone se3.05h06 and the *Citullus lanatus* cysteine synthase. The soybean sequence is 42% identical to the *Citullus lanatus* over 325 amino acids.

The BLASTX search using the nucleotide sequences of clones cenl.pk0061.d4, r1r12.pk0026.g1, sf11.pk0012.c4. and wr1.pk0091.g6 revealed similarity of the proteins encoded by the cDNAs to *Arabidopsis thaliana* cystathionine β-lyase (GenBank Accession No. L40511). The BLAST results for each of these ESTs are shown in TABLE 6:

**TABLE 6**

| BLAST Results for Clones Encoding Polypeptides Homologous to Cystathionine β-Lyase | |
|---|---|
| | BLAST pLog Score |
| Clone | GenBank Accession No L40511 |
| cen1.pk0061.d4 | 50.41 |
| r1r12.pk0026.g1 | 39.00 |
| sf11.pk0012.c4 | 33.85 |
| wr1.pk0091.g6 | 52.52 |

The sequence of the entire cDNA insert in clone cen1.pk0061.d4 was determined and is set forth in SEQ ID NO:33; the deduced amino acid sequence of this cDNA is shown in SEQ ID NO:34. The entire cDNA insert in clone cen1.pk0061.d4 was reevaluated by BLAST, yielding an even higher pLog value vs. the *Arabidopsis thaliana* (L40511; pLog = 128.54) enzyme. The sequence of a portion of the cDNA insert in clone r1r12.pk0026.g1 is set forth in SEQ ID NO:35; the deduced amino acid sequence of this cDNA is shown in SEQ ID NO:36. The sequence of the entire cDNA insert in clone sf11.pk0012.c4 was determined and is set forth in SEQ ID NO:37; the deduced amino acid sequence of this cDNA is shown in SEQ ID NO:38. The entire cDNA insert in clone sf11.pk0012.c4 was reevaluated by BLAST, yielding an even higher pLog value vs. the *Arabidopsis thaliana* (L40511; pLog = 221.92) enzyme. The sequence of a portion of the cDNA insert in clone wr1.pk0091.g6 is set forth in SEQ ID NO:39; the deduced amino acid sequence of this cDNA is shown in SEQ ID NO:40. Sequence alignments and BLAST scores and probabilities indicate that the instant nucleic acid fragments encode a portion of a corn (cen1.pk0061.d4), rice (r1r12.pk0026.g1), and wheat (wrl.pk0091.g6) cystathionine β-lyase enzyme. The clone sf11.pk0012.c4 encodes an entire soybean cystathionine β-lyase enzyme. These are the first corn, rice, soybean, and wheat ESTs identified that encode a cystathionine β-lyase enzyme. Figure 6 shows the alignment of the deduced amino acid sequence from clone cen1.pk0061.d4, r1r12.pk0026.g1, wr1.pk0091.g6, sf11.pk0012.c4 with the *Arabidopsis thaliana* cystathionine β-lyase sequence. Over 226 amino acids the corn sequence (cen1.pk0061.d4) is 88.5% identical to the soybean (sf11.pk0012.c4) sequence and 81% identical to the *Arabidopsis thaliana* sequence. The rice sequence contains 75 amino acids which are 68% identical to the soybean sequence, and 76% identical to the *Arabidopsis thaliana* sequence. The wheat sequence contains 42 amino acids which are 66.41 % identical to the soybean sequence and 71% identical to the *Arabidopsis thaliana* sequence. The 465 amino acid soybean sequence (sf11.pk0012.c4) is 74% identical to the *Arabidopsis thaliana* sequence.

### EXAMPLE 3

### Expression of Chimeric Genes in Monocot Cells

A chimeric gene comprising a cDNA encoding an amino acid biosynthetic enzyme in sense orientation with respect to the maize 27 kD zein promoter that is located 5' to the cDNA fragment, and the 10 kD zein 3' end that is located 3' to the cDNA fragment, can be constructed. The cDNA fragment of this gene may be generated by polymerase chain reaction (PCR) of the cDNA clone using appropriate oligonucleotide primers. Cloning sites (NcoI or SmaI) can be incorporated into the oligonucleotides to provide proper orientation of the DNA fragment when inserted into the digested vector pML103 as described below. Amplification is then performed in a standard PCR. The amplified DNA is then digested with restriction enzymes NcoI and SmaI and fractionated on an agarose gel. The appropriate band can be isolated from the gel and combined with a 4.9 kb Ncol-Smal fragment of the plasmid pML103. Plasmid pML103 has been deposited under the terms of the Budapest Treaty at ATCC (American Type Culture Collection, 10801 University Boulevard, Manassas, VA 20110-2209), and bears accession number ATCC 97366. The DNA segment from pML103 contains a 1.05 kb SalI-NcoI promoter fragment of the maize 27 kD zein gene and a 0.96 kb SmaI-SalI fragment from the 3' end of the maize 10 kD zein gene in the vector pGem9Zf(+) (Promega). Vector and insert DNA can be ligated at 15°C overnight, essentially as described (Maniatis). The ligated DNA may then be used to transform *E. coli* XL1-Blue (Epicurian Coli XL-1 Blue™; Stratagene). Bacterial transformants can be screened by restriction enzyme digestion of plasmid DNA and limited nucleotide sequence analysis using the dideoxy chain termination method (Sequenase^{™} DNA Sequencing Kit; U. S. Biochemical). The resulting plasmid construct would comprise a chimeric gene encoding, in the 5' to 3' direction, the maize 27 kD zein promoter, a cDNA fragment encoding a plant amino acid biosynthetic enzyme, and the 10 kD zein 3' region.

The chimeric gene described above can then be introduced into corn cells by the following procedure. Immature corn embryos can be dissected from developing caryopses derived from crosses of the inbred corn lines H99 and LH132. The embryos are isolated 10 to 11 days after pollination when they are 1.0 to 1.5 mm long. The embryos are then placed with the axis-side facing down and in contact with agarose-solidified N6 medium (Chu et al., (1975) Sci. Sin. Peking 18:659-668). The embryos are kept in the dark at 27°C. Friable embryogenic callus consisting of undifferentiated masses of cells with somatic proembryoids and embryoids borne on suspensor structures proliferates from the scutellum of these immature embryos. The embryogenic callus isolated from the primary explant can be cultured on N6 medium and sub-cultured on this medium every 2 to 3 weeks.

The plasmid, p35S/Ac (obtained from Dr. Peter Eckes. Hoechst Ag, Frankfurt, Germany) may be used in transformation experiments in order to provide for a selectable marker. This plasmid contains the *Pat* gene (see European Patent Publication 0 242 236) which encodes phosphinothricin acetyl transferase (PAT). The enzyme PAT confers resistance to herbicidal glutamine synthetase inhibitors such as phosphinothricin. The *pat* gene in p35S/Ac is under the control of the 35S promoter from Cauliflower Mosaic Virus (Odell et al. (1985) Nature 313:810-812) and the 3' region of the nopaline synthase gene from the T-DNA of the Ti plasmid of *Agrohaclerium tumefaciens.*

The particle bombardment method (Klein et al., (1987) Nature 327:70-73) may be used to transfer genes to the callus culture cells. According to this method, gold particles (1 µm in diameter) are coated with DNA using the following technique. Ten µg of plasmid DNAs are added to 50 µL of a suspension of gold particles (60 mg per mL). Calcium chloride (50 µL of a 2.5 M solution) and spermidine free base (20 µL of a 1.0 M solution) are added to the particles. The suspension is vortexed during the addition of these solutions. After 10 minutes, the tubes are briefly centrifuged (5 sec at 15,000 rpm) and the supernatant removed. The particles are resuspended in 200 µL of absolute ethanol, centrifuged again and the supernatant removed. The ethanol rinse is performed again and the particles resuspended in a final volume of 30 µL of ethanol. An aliquot (5 µL) of the DNA-coated gold particles can be placed in the center of a Kapton^{™} flying disc (Bio-Rad Labs). The particles are then accelerated into the corn tissue with a Biolistic^{™} PDS-1000/He (Bio-Rad Instruments, Hercules CA), using a helium pressure of 1000 psi, a gap distance of 0.5 cm and a flying distance of 1.0 cm.

For bombardment, the embryogenic tissue is placed on filter paper over agarose-solidified N6 medium. The tissue is arranged as a thin lawn and covered a circular area of about 5 cm in diameter. The petri dish containing the tissue can be placed in the chamber of the PDS-1000/He approximately 8 cm from the stopping screen. The air in the chamber is then evacuated to a vacuum of 28 inches of Hg. The macrocarrier is accelerated with a helium shock wave using a rupture membrane that bursts when the He pressure in the shock tube reaches 1000 psi.

Seven days after bombardment the tissue can be transferred to N6 medium that contains gluphosinate (2 mg per liter) and lacks casein or proline. The tissue continues to grow slowly on this medium. After an additional 2 weeks the tissue can be transferred to fresh N6 medium containing gluphosinate. After 6 weeks, areas of about 1 cm in diameter of actively growing callus can be identified on some of the plates containing the glufosinate-supplemented medium. These calli may continue to grow when sub-cultured on the selective medium.

Plants can be regenerated from the transgenic callus by first transferring clusters of tissue to N6 medium supplemented with 0.2 mg per liter of 2,4-D. After two weeks the tissue can be transferred to regeneration medium (Fromm et al., (1990) Bio/Technology 8:833-839).

### EXAMPLE 4

### Expression of Chimeric Genes in Dicot Cells

A seed-specific expression cassette composed of the promoter and transcription terminator from the gene encoding the β subunit of the seed storage protein phaseolin from the bean *Phaseolus vulgaris* (Doyle et al. (1986) J. Biol. Chem. 261:9228-9238) can be used for expression of the instant amino acid biosynthetic enzymes in transformed soybean. The phaseolin cassette includes about 500 nucleotides upstream (5') from the translation initiation codon and about 1650 nucleotides downstream (3') from the translation stop codon of phaseolin. Between the 5' and 3' regions are the unique restriction endonuclease sites Nco I (which includes the ATG translation initiation codon), Sma I, Kpn I and Xba I. The entire cassette is flanked by Hind III sites.

The cDNA fragment of this gene may be generated by polymerase chain reaction (PCR) of the cDNA clone using appropriate oligonucleotide primers. Cloning sites can be incorporated into the oligonucleotides to provide proper orientation of the DNA fragment when inserted into the expression vector. Amplification is then performed as described above, and the isolated fragment is inserted into a pUC18 vector carrying the seed expression cassette.

Soybean embroys may then be transformed with the expression vector comprising sequences encoding a plant amino acid biosynthetic enzyme. To induce somatic embryos, cotyledons, 3-5 mm in length dissected from surface sterilized, immature seeds of the soybean cultivar A2872, can be cultured in the light or dark at 26°C on an appropriate agar medium for 6-10 weeks. Somatic embryos which produce secondary embryos are then excised and placed into a suitable liquid medium. After repeated selection for clusters of somatic embryos which multiplied as early, globular staged embryos, the suspensions are maintained as described below.

Soybean embryogenic suspension cultures can maintained in 35 mL liquid media on a rotary shaker, 150 rpm, at 26°C with florescent lights on a 16:8 hour day/night schedule. Cultures are subcultured every two weeks by inoculating approximately 35 mg of tissue into 35 mL of liquid medium.

Soybean embryogenic suspension cultures may then be transformed by the method of particle gun bombardment (Kline et al. (1987) Nature (London) 327:70, U.S. Patent No. 4,945,050). A Du Pont Biolistic^{™} PDS 1000/HE instrument (helium retrofit) can be used for these transformations.

A selectable marker gene which can be used to facilitate soybean transformation is a chimeric gene composed of the 35S promoter from Cauliflower Mosaic Virus (Odell et al. (1985) Nature 313:810-812), the hygromycin phosphotransferase gene from plasmid pJR225 (from *E. coli*; Gritz et al. (1983) Gene 25:179-188) and the 3' region of the nopaline synthase gene from the T-DNA of the Ti plasmid of *Agrobacterium tumefaciens*. The seed expression cassette comprising the phaseolin 5' region, the fragment encoding the biosynthetic enzyme and the phaseolin 3' region can be isolated as a restriction fragment. This fragment can then be inserted into a unique restriction site of the vector carrying the marker gene.

To 50 µL of a 60 mg/mL 1 µm gold particle suspension is added (in order): 5 µL DNA (1 µg/µL), 20 µl spermidine (0.1 M), and 50 µL CaCl₂ (2.5 M). The particle preparation is then agitated for three minutes, spun in a microfuge for 10 seconds and the supernatant removed. The DNA-coated particles are then washed once in 400 µL 70% ethanol and resuspended in 40 µL of anhydrous ethanol. The DNA/particle suspension can be sonicated three times for one second each. Five µL of the DNA-coated gold particles are then loaded on each macro carrier disk.

Approximately 300-400 mg of a two-week-old suspension culture is placed in an empty 60x15 mm petri dish and the residual liquid removed from the tissue with a pipette. For each transformation experiment, approximately 5-10 plates of tissue are normally bombarded. Membrane rupture pressure is set at 1100 psi and the chamber is evacuated to a vacuum of 28 inches mercury. The tissue is placed approximately 3.5 inches away from the retaining screen and bombarded three times. Following bombardment, the tissue can be divided in half and placed back into liquid and cultured as described above.

Five to seven days post bombardment, the liquid media may be exchanged with fresh media, and eleven to twelve days post bombardment with fresh media containing 50 mg/mL hygromycin. This selective media can be refreshed weekly. Seven to eight weeks post bombardment, green, transformed tissue may be observed growing from untransformed, necrotic embryogenic clusters. Isolated green tissue is removed and inoculated into individual flasks to generate new, clonally propagated, transformed embryogenic suspension cultures. Each new line may be treated as an independent transformation event. These suspensions can then be subcultured and maintained as clusters of immature embryos or regenerated into whole plants by maturation and germination of individual somatic embryos.

### EXAMPLE 5

### Analysis of Amino Acid Content of the Seeds of Transformed Plants

To analyze for expression of the chimeric genes in seeds and for the consequences of expression on the amino acid content in the seeds, a seed meal can be prepared by any of a number of suitable methods known to those skilled in the art. The seed meal can be partially or completely defatted, via hexane extraction for example, if desired. Protein extracts can be prepared from the meal and analyzed for enzyme activity. Alternatively the presence of any of the expressed enzymes can be tested for immunologically by methods well-known to those skilled in the art. To measure free amino acid composition of the seeds, free amino acids can be extracted from the meal and analyzed by methods known to those skilled in the art (Bieleski et al. (1966) Anal. Biochem. 17:278-293). Amino acid composition can then be determined using any commercially available amino acid analyzer. To measure total amino acid composition of the seeds, meal containing both protein-bound and free amino acids can be acid hydrolyzed to release the protein-bound amino acids and the composition can then be determined using any commercially available amino acid analyzer. Seeds expressing the instant amino acid biosynthetic enzymes and with altered lysine, threonine, methionine, cysteine and/or isoleucine content than the wild type seeds can thus be identified and propagated.

To measure free amino acid composition of the seeds, free amino acids can be extracted from 8-10 milligrams of the seed meal in 1.0 mL of methanol/chloroform/water mixed in ratio of 12v/5v/3v (MCW) at room temperature. The mixture can be vortexed and then centrifuged in an eppendorf microcentrifuge for about 3 min; approximately 0.8 mL of supernatant is then decanted. To this supernatant, 0.2 mL of chloroform is added followed by 0.3 mL of water. The mixture is then vortexed and centrifuged in an eppendorf microcentrifuge for about 3 min. The upper aqueous phase, approximately 1.0 mL, can then be removed and dried down in a Savant Speed Vac Concentrator. The samples are then hydrolyzed in 6N hydrochloric acid, 0.4% β-mercaptoethanol under nitrogen for 24 h at 110-120°C. Ten percent of the sample can then be analyzed using a Beckman Model 6300 amino acid analyzer using post-column ninhydrin detection. Relative free amino acid levels in the seeds are then compared as ratios of lysine, threonine, methionine, cysteine and/or isoleucine to leucine, thus using leucine as an internal standard.

### EXAMPLE 6

### Expression of Chimeric Genes in Microbial Cells

The cDNAs encoding the instant plant amino acid biosynthetic enzymes can be inserted into the T7 *E. coli* expression vector pBT430. This vector is a derivative of pET-3a (Rosenberg et al. (1987) Gene 56:125-135) which employs the bacteriophage T7 RNA polymerase/T7 promoter system. Plasmid pBT430 was constructed by first destroying the EcoR I and Hind III sites in pET-3a at their original positions. An oligonucleotide adaptor containing EcoR I and Hind III sites was inserted at the BamH I site of pET-3a. This created pET-3aM with additional unique cloning sites for insertion of genes into the expression vector. Then, the Nde I site at the position of translation initiation was converted to an Nco I site using oligonucleotide-directed mutagenesis. The DNA sequence of pET-3aM in this region, 5'-CATATGG, was converted to 5'-CCCATGG in pBT430.

Plasmid DNA containing a cDNA may be appropriately digested to release a nucleic acid fragment encoding the enzyme. This fragment may then be purified on a 1% NuSieve GTG™ low melting agarose gel (FMC). Buffer and agarose contain 10 µg/ml ethidium bromide for visualization of the DNA fragment. The fragment can then be purified from the agarose gel by digestion with GELase™ (Epicentre Technologies) according to the manufacturer's instructions, ethanol precipitated, dried and resuspended in 20 µL of water. Appropriate oligonucleotide adapters may be ligated to the fragment using T4 DNA ligase (New England Biolabs. Beverly, MA). The fragment containing the ligated adapters can be purified from the excess adapters using low melting agarose as described above. The vector pBT430 is digested, dephosphorylated with alkaline phosphatase (NEB) and deproteinized with phenol/chloroform as described above. The prepared vector pBT430 and fragment can then be ligated at 16°C for 15 hours followed by transformation into DH5 electrocompetent cells (GIBCO BRL). Transformants can be selected on agar plates containing LB media and 100 µg/mL ampicillin. Transformants containing the gene encoding the enzyme are then screened for the correct orientation with respect to the T7 promoter by restriction enzyme analysis.

For high level expression, a plasmid clone with the cDNA insert in the correct orientation relative to the T7 promoter can be transformed into *E. coli* strain BL21(DE3) (Studier et al. (1986) J. Mol. Biol. 189:113-130). Cultures are grown in LB medium containing ampicillin (100 mg/L) at 25°C. At an optical density at 600 nm of approximately 1, IPTG (isopropylthio-β-galactoside, the inducer) can be added to a final concentration of 0.4 mM and incubation can be continued for 3 h at 25°. Cells are then harvested by centrifugation and re-suspended in 50 µL of 50 mM Tris-HCl at pH 8.0 containing 0.1 mM DTT and 0.2 mM phenyl methylsulfonyl fluoride. A small amount of 1 mm glass beads can be added and the mixture sonicated 3 times for about 5 seconds each time with a microprobe sonicator. The mixture is centrifuged and the protein concentration of the supernatant determined. One µg of protein from the soluble fraction of the culture can be separated by SDS-polyacrylamide gel electrophoresis. Gels can be observed for protein bands migrating at the expected molecular weight.

### EXAMPLE 7

### Evaluating Compounds for Their Ability to Inhibit the Activity of a Plant Amino Acid Biosynthetic Enzyme

The plant amino acid biosynthetic enzymes described herein may be produced using any number of methods known to those skilled in the art. Such methods include, but are not limited to, expression in bacteria as described in Example 6, or expression in eukaryotic cell culture, *in planta*, and using viral expression systems in suitably infected organisms or cell lines. The instant enzymes may be expressed separately as mature proteins, or may be co-expressed in *E. coli* or another suitable expression background. In addition, whether expressed separately or in combination, the instant enzymes may be expressed either as mature forms of the proteins as observed *in vivo* or as fusion proteins by covalent attachment to a variety of enzymes, proteins or affinity tags. Common fusion protein partners include glutathione S-transferase ("GST"), thioredoxin ("Trx"), maltose binding protein, and C-and/or N-terminal hexahistidine polypeptide ("(His)₆"). The fusion proteins may be engineered with a protease recognition site at the fusion point so that fusion partners can be separated by protease digestion to yield intact mature enzymes. Examples of such proteases include thrombin, enterokinase and factor Xa. However, any protease can be used which specifically cleaves the peptide connecting the fusion protein and the biosynthetic enzyme.

Purification of the instant enzymes, if desired, may utilize any number of separation technologies familiar to those skilled in the art of protein purification. Examples of such methods include, but are not limited to, homogenization, filtration, centrifugation, heat denaturation, ammonium sulfate precipitation, desalting, pH precipitation, ion exchange chromatography, hydrophobic interaction chromatography and affinity chromatography, wherein the affinity ligand represents a substrate, substrate analog or inhibitor. When the enzymes are expressed as fusion proteins, the purification protocol may include the use of an affinity resin which is specific for the fusion protein tag attached to the expressed enzyme or an affinity resin containing ligands which are specific for the enzyme. For example, an enzyme may be expressed as a fusion protein coupled to the C-terminus of thioredoxin. In addition, a (His)₆ peptide may be engineered into the N-terminus of the fused thioredoxin moiety to afford additional opportunities for affinity purification. Other suitable affinity resins could be synthesized by linking the appropriate ligands to any suitable resin such as Sepharose-4B. In an alternate embodiment, a thioredoxin fusion protein may be eluted using dithiothreitol; however, elution may be accomplished using other reagents which interact to displace the thioredoxin from the resin. These reagents include β-mercaptoethanol or other reduced thiol. The eluted fusion protein may be subjected to further purification by traditional means as stated above, if desired. Proteolytic cleavage of the thioredoxin fusion protein and the biosynthetic enzyme may be accomplished after the fusion protein is purified or while the protein is still bound to the ThioBond™ affinity resin or other resin.

Crude, partially purified or purified enzyme, either alone or as a fusion protein, may be utilized in assays for the evaluation of compounds for their ability to inhibit enzymatic activation of the plant amino acid biosynthetic enzymes disclosed herein. Assays may be conducted under well known experimental conditions which permit optimal enzymatic activity. Examples of assays for many of these enzymes can be found in Methods in Enzymology Vol. V, (Colowick and Kaplan eds.) Academic Press, New York or Methods in Enzymology Vol. XVII, (Tabor and Tabor eds.) Academic Press; New York. Specific examples may be found in the following references, aspartic semialdehyde dehydrogenase may be assayed as described in Black et al. (1955) J. Biol. Chem. 213:39-50, or Cremer et al. (1988) J. Gen. Microbiol. 134:3221-3229; diaminopimelate decarboxylase may be assayed as described in Work (1962) in Methods in Enzymology Vol. V, (Colowick and Kaplan eds.) 864-870, Academic Press, New York or Cremer et al. (1988) J. Gen. Microbiol. 134:3221-3229; homoserine kinase may be assayed as described in Aames (1976) Plant Sci. Lett. 7:187-194; cysteine synthase may be assayed as described in Thompson et al. (1968) Biochem. Biophys. Res. Common. 31: 281-286 or Bertagnolli et al. (1977) Plant Physiol. 60:115-121; and cystathionine β-lyase may be assayed as described in Giovanelli et al. (1971) Biochim. Biophys. Acta 227:654-670 or Droux et al. (1995) Arch. Biochem Biophys. 316:585-595.

## Claims

1. An isolated nucleic acid fragment encoding a plant cysteine synthase comprising a member selected from the group consisting of:
(a) an isolated nucleic acid fragment encoding the amino acid sequence set forth in SEQ ID NO:31;
(b) an isolated nucleic acid fragment that is at least 90% identical to a nucleic acid fragment encoding the amino acid sequence set forth in SEQ ID NO:31;
(c) an isolated nucleic acid fragment that is complementary to (a) or (b).

2. The isolated nucleic acid fragment of Claim 1 wherein the nucleotide sequence of the fragment comprises SEQ ID NO:30.

3. A chimeric gene comprising the nucleic acid fragment of Claim 1 or Claim 2 operably linked to regulatory sequences which influence the transcription, RNA processing or stability, or translation of the coding sequence.

4. A transformed host cell comprising the chimeric gene of Claim 3.

5. A cysteine synthase polypeptide comprising an amino acid sequence set forth in SEQ ID NO:31.

6. A method of obtaining a nucleic acid fragment encoding the amino acid sequence encoding a plant cysteine synthase comprising:
(a) probing a cDNA or genomic library with the nucleic acid fragment of Claim 1;
(b) identifying a DNA clone that hybridizes with the nucleic acid fragment of Claim 1;
(c) isolating the DNA clone identified in step (b); and
(d) sequencing the cDNA or genomic fragment that comprises the clone isolated in step (c)
wherein the sequenced nucleic acid fragment encodes the amino acid sequence encoding a plant cysteine synthase.

## Patentansprüche

1. Isoliertes Nucleinsäurefragment, codierend eine Pflanzen-Cystein-Synthase, umfassend einen Bestandteil, ausgewählt aus der Gruppe, bestehend aus:
(a) einem isolierten Nucleinsäurefragment, codierend die Aminosäuresequenz, angegeben in SEQ ID NO:31;
(b) einem isolierten Nucleinsäurefragment, das zu mindestens 90% identisch mit einem Nucleinsäurefragment, codierend die Aminosäuresequenz, angegeben in SEQ ID NO:31, ist;
(c) einem isolierten Nucleinsäurefragment, das komplementär zu (a) oder (b) ist.

2. Isoliertes Nucleinsäurefragment nach Anspruch 1, wobei die Nucleotidsequenz des Fragments SEQ ID NO:30 umfaßt.

3. Chimäres Gen, umfassend das Nucleinsäurefragment nach Anspruch 1 oder Anspruch 2, operabel verknüpft mit regulatorischen Sequenzen, welche die Transkription, RNA-Verarbeitung oder -Stabilität oder Translation der codierenden Sequenz beeinflussen.

4. Transformierte Wirtszelle, umfassend das chimäre Gen nach Anspruch 3.

5. Cystein-Synthase-Polypeptid, umfassend eine Aminosäuresequenz, angegeben in SEQ ID NO:31.

6. Verfahren zum Erhalt eines Nucleinsäurefragments, codierend die Aminosäuresequenz, codierend eine Pflanzen-Cystein-Synthase, umfassend:
(a) Sondieren einer cDNA- oder genomischen Bibliothek mit dem Nucleinsäurefragment nach Anspruch 1;
(b) Identifizieren eines DNA-Klons, der mit dem Nucleinsäurefragment nach Anspruch 1 hybridisiert;
(c) Isolieren des DNA-Klons, identifiziert in Schritt (b); und
(d) Sequenzieren des cDNA- oder genomischen Fragments, das den Klon, isoliert in Schritt (c), umfaßt,
wobei das sequenzierte Nucleinsäurefragment die Aminosäuresequenz, codierend eine Pflanzen-Cystein-Synthase, codiert.

## Revendications

1. Fragment d'acide nucléique isolé codant une cystéine synthase végétale, comprenant un membre sélectionné parmi le groupe consistant en:
(a) un fragment d'acide nucléique isolé codant la séquence d'acides aminés présentée dans la SEQ ID NO: 31;
(b) un fragment d'acide nucléique isolé qui est au moins identique à 90% à un fragment d'acide nucléique codant la séquence d'acides aminés présentée dans la SEQ ID NO: 31;
(c) un fragment d'acide nucléique isolé qui est complémentaire à (a) ou (b).

2. Le fragment d'acide nucléique isolé de la revendication 1, dans lequel la séquence de nucléotides du fragment comprend la SEQ ID NO: 30.

3. Gène chimérique comprenant le fragment d'acide nucléique de la revendication 1 ou de la revendication 2, lié de manière opérationnelle à des séquences régulatrices qui influencent la transcription, l'élaboration ou la stabilité de l'ARN ou la traduction de la séquence codante.

4. Cellule hôte transformée comprenant le gène chimérique de la revendication 3.

5. Polypeptide de cystéine synthase comprenant une séquence d'acides aminés présentée dans la SEQ ID NO: 31.

6. Procédé d'obtention d'un fragment d'acide nucléique codant la séquence d'acides aminés codant une cystéine synthase végétale, comprenant:
(a) sonder une banque d'ADNc ou génomique avec le fragment d'acide nucléique de la revendication 1;
(b) identifier un clone d'ADN qui s'hybride avec le fragment d'acide nucléique de la revendication 1;
(c) isoler le clone d'ADN identifié à l'étape (b) et
(d) séquencer l'ADNc ou le fragment génomique qui comprend le clone isolé à l'étape (c),
où le fragment d'acide nucléique séquence code la séquence d'acides aminés codant une cystéine synthase végétale.


### SEQUENCE LISTING

(1) GENERAL INFORMATION:
   (i) APPLICANT:
      (A) ADDRESSEE: E. I. DU PONT DE NEMOURS AND CCMPANY
      (B) STREET: 1007 MARKET STREET
      (C) CITY: WILMINGTON
      (D) STATE: DELAWARE
      (E) COUNTRY: USA
      (F) ZIP: 19898
      (G) TELEPHONE: 302-992-4926
      (H) TELEFAX: 302-773-0164
      (I) TELEX: 6717325
   (ii) TITLE OF INVENTION: PLANT AMINO ACID BIOSYNTHETIC ENZYMES
   (iii) NUMBER OF SEQUENCES: 41
   (iv) COMPUTER READABLE FORM:
      (A) MEDIUM TYPE: DISKETTE, 3.50 INCH
      (B) COMPUTER: IBM PC COMPATIBLE
      (C) OPERATING SYSTEM: MICROSOFT WORD FOR WINDOWS 95
      (D) SOFTWARE: MICROSOFT WORD VERSION 7.0A
   (v) CURRENT APPLICATION DATA:
      (A) APPLICATION NUMBER:
      (B) FILING DATE:
      (C) CLASSIFICATION:
   (vi) PRIOR APPLICATION DATA:
      (A) APPLICATION NUMBER: 60/049,406
      (B) FILING DATE: JUNE 12, 1997
   (vii) ATTORNEY/AGENT INFORMATION:
      (A) NAME: MAJARIAN, WILLIAM R.
      (B) REGISTRATION NUMBER: 41,173
      (C) REFERENCE/DOCKET NUMBER: BB-1116
(2) INFORMATION FOR SEQ ID NO:1:
   (i) SEQUENCE CHARACTERISTICS:
      (A) LENGTH: 826 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: r1r48.pk003.d12
   (xi) SEQUENCE DESCRIPTION: SEQ ID NO:1:
   TGGTACCGCC ACGCCAAGGT GGTAAGGATG GTTGTCAGCA CTTACCAAGC AGCAAGTGGT    60
   GCTGGGGCTG CGGCCATGGA AGAACTCAAA CTTCAAACTC AAGAGGTCTT GGCGGGGAAA   120
   GCACCAACAT GCAACATTTT CAGTCAGCAG TATGCTTTTA ATATATTTTC ACATAATGCA   180
   CCAATTGTTG AAAATGGGTA CAATGAGGAG GAGATGAAGA TGGTGAAGGA GACCAGAAAA   240
   ATCTGGAATG ATAAAGATGT GAAGGTAACT GCAACCTGCA TACGAGTTCC TGTGATGCGT   300
   GCACATGCTG AAAGTGTGAA TCTACAGTTT GAAAAGCCAC TTGATGAGGA TACTGCAAGG   360
   GAAATCTTGA GGGCAGCTGA AGGTGTTACC ATTATTGATG ACCGTGCTTC CAATCGCTTC   420
   CCCACACCTC TTGAGGTATC GGATAAAGAT GATGTAGCAG TGGGTAGAAT TCGTCAGGAT   480
   TTGTCGCAAG ATGATAACAA AGGGCTGCAC ATATTTGTTT GTGGAGATCA AATACGTAAA   540
   GGTGCTGCAC TCAATGCTGT GCAGATTGCT GAAATGCTAC TCAAGTGATT TTCTTTTCTG   600
   TACCTTTCTC TCCTTGCCCC TCTTTGCTCT AGTCATTGTT TGACGGATGT ACTCTGGTTA   660
   GTATGAGATC AATTTTGATC ATCTTTTGTA ATCTATATTC CTAGTGAAAT AAATGTAAAA   720
   CGGTTTTGCT CTATCTTCTG CACAAGTGTA GAAGAAATCT GAAATTGGGA AATTGGAGTG   780
   TGGCCCTTGT TCAAAAAAAA AAAAAAAAAA AAAAAAAAAA AAAAAA                  826
(2) INFORMATION FOR SEQ ID NO:2:
   (i) SEQUENCE CHARACTERISTICS:
      (A) LENGTH: 195 amino acids
      (B) TYPE: amino acid
      (C) STRANDEDNESS: not relevant
      (D) TOPOLOGY: linear
   (ii) MOLECULE TYPE peptide
   (vii) IMMEDIATE SOURCE:
      (B) CLONE: r1r48.pk003.d12
   (xi) SEQUENCE DESCRIPTION: SEQ ID NO:2:
   Trp Tyr Arg His Ala Lys Val Val Arg Met Val Val Ser Thr Tyr Gln
   1               5                   10                  15
   Ala Ala Ser Gly Ala Gly Ala Ala Ala Met Glu Glu Leu Lys Leu Gln
               20                  25                  30
   Thr Gln Glu Val Leu Ala Gly Lys Ala Pro Thr Cys Asn Ile Phe Ser
           35                  40                  45
   Gln Gln Tyr Ala Phe Asn Ile Phe Ser His Asn Ala Pro Ile Val Glu
       50                  55                  60
   Asn Gly Tyr Asn Glu Glu Glu Met Lys Met Val Lys Glu Thr Arg Lys
   65                  70                  75                  80
   Ile Trp Asn Asp Lys Asp Val Lys Val Thr Ala Thr Cys Ile Arg Val
                   85                  90                  95
   Pro Val Met Arg Ala His Ala Glu Ser Val Asn Leu Gln Phe Glu Lys
               100                 105                 110
   Pro Leu Asp Glu Asp Thr Ala Arg Glu Ile Leu Arg Ala Ala Glu Gly
           115                 120                 125
   Val Thr Ile Ile Asp Asp Arg Ala Ser Asn Arg Phe Pro Thr Pro Leu
       130                 135                 140
   Glu Val Ser Asp Lys Asp Asp Val Ala Val Gly Arg Ile Arg Gln Asp
   145                 150                 155                 160
   Leu Ser Gln Asp Asp Asn Lys Gly Leu Asp Ile Phe Val Cys Gly Asp
                   165                 170                 175
   Gln Ile Arg Lys Gly Ala Ala Leu Asn Ala Val Gln Ile Ala Glu Met
               180                 185                 190
   Leu Leu Lys
           195
(2) INFORMATION FOR SEQ ID NO:3:
   (i) SEQUENCE CHARACTERISTICS:
      (A) LENGTH: 875 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: wr1.pk0004.c11
   (xi) SEQUENCE DESCRIPTION: SEQ ID NO:3:
   CCTCATGGCT GTCACGCCGC TGCATCGCCA CGCCAAGGTG AAAAGGATGG TTGTCAGCAC    60
   ATACCAAGCA GCAAGTGGTG CTGGTGCTGC AGCCATGGAA GAACTCAAAC TTCAGACTCG   120
   AGAGGTCTTG GAAGGAAAGC CACCAACCTG TAACATTTTC AGTCAACAGT ATGCTTTTAA   180
   TATATTTTCG CATAATGCAC CTATTGTTGA AAATGGCTAT AATGAGGAAG AGATGAAAAT   240
   GGTGAAGGAG ACCAGAAAAA TCTGGAATGA CAAGGATGTA AGAGTAACTG CAACTTGTAT   300
   ACGGGTTCCT ACGATGCGCG CGCATGCCGA AAGCGTGAAT CTACAGTTTG AAAAGCCACT   360
   TGATGAGGAC ACTGCCAGAG AAATCTTGAG GGCAGCTCCT GGTGTTACCA TTAGTGACGA   420
   CCGTGCTGCC AACCGCTTCC CTACACCACT GGAGGTATCG GATAAAGATG ACGTATCAGT   480
   TGGTAGGATT CGCCAGGACT TGTCACAAGA TGATAACAGA GGGTTGGAGT TATTTGTCTG   540
   TGGAGACCAG ATACGTAAAG GCGCCGCGCT GAACGCTGTG CAGATTGCTG AAATGCTACT   600
   GAAGTGACCG CCTTTTTACC ATTGTCTCAT GTGCCACGTT GCTCTATCCA TTGATGGATT   660
   GATGTACTCT AGTCACTTTC AACCCAGTTT TGGTCGTCGT CTTTTTTGTA ATCTGTCAAC   720
   CTAGCAGAAG AAGTGTAAGA CGGGCTTTAG TCATCTGTTG CACACAAAAG TGCAGCCACA   780
   AGTTTAGAAA AGGAGGGTTT TCACTTGTTC GGATTTTGCC TTAGGTTGGA CTTTGTTGCA   840
   AGTTGTCGTT TGTTTCTTGA AAGCTGGTCT GCTGT                              875

   (i) SEQUENCE CHARACTERISTICS:
      (A) LENGTH: 201 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: wr1.pk0004.c11
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 457 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: sfll.pk0122.f9
   (xi) SEQUENCE DESCRIPTION: SEQ ID NO:5:
   GTCTGTTTTA AAATCCAACA CTTAATCTCT CTCTTCGCAG CCTAAAATCC CAATGGCTTC    60
   ACTCTCTGTT TTGCGCCACA ACCACCTCTT CTCGGGCCCC CTCCCGGCCC GCCCCAAGCC   120
   CACCTCCTCC TCCTCCTCCA GGATCCGAAT GTCCCTCCGC GAGAACGGCC CCTCCATCGC   180
   CGTCGTGGGC GTCACCGGCG CCGTCGGCCA NGAGTTCCTC TCCGTCCTCT CCGACCGCGA   240
   CTTCCCCTAC CGCTCCATTC ATATGCTGGC TTCCAAGCGC TCCGCTGGAC GCCGCATCAC   300
   CTTCGAGGAC AGGGACTACN TCTTCAGGAG CTCACGCCGG AGAGTTCGAC GGTGTCGACA   360
   TCGCGCTCTT CAGCGCNGGG GGTCCATCAA NNAAGCATTC GGACCATCGN CGTAAATCGN   420
   GGGACGGNCG TNGNCAANAT ANCTCCGGTT NCCTTTG                            457
(2) INFORMATION FOR SEQ ID NO:6:
   (i) SEQUENCE CHARACTERISTICS:
      (A) LENGTH: 86 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: sf11.pk0122.f9
   (xi) SEQUENCE DESCRIPTION: SEQ ID NO:6:
   Met Ala Ser Leu Ser Val Leu Arg His Asn His Leu Phe Ser Gly Pro
   1               5                   10                  15
   Leu Pro Ala Arg Pro Lys Pro Thr Ser Ser Ser Ser Ser Arg Ile Arg
               20                  25                  30
   Met Ser Leu Arg Glu Asn Gly Pro Ser Ile Ala Val Val Gly Val Thr
           35                  40                  45
   Gly Ala Val Gly Gln Glu Phe Leu Ser Val Leu Ser Asp Arg Asp Phe
       50                  55                  60
   Pro Tyr Arg Ser Ile His Met Leu Ala Ser Lys Arg Ser Ala Gly Arg
   65                  70                  75                  80
   Arg Ile Thr Phe Glu Asp
                   85
(2) INFORMATION FOR SEQ ID NO:7:
   (i) SEQUENCE CHARACTERISTICS:
      (A) LENGTH: 160 amino acids
      (B) TYPE: amino acid
      (C) STRANDEDNESS: single
      (D) TOPOLOGY: linear
   (ii) MOLECULE TYPE: cDNA
   (vi) ORIGINAL SOURCE:
      (A) ORGANISM: Legionella pneumophila
   (xi) SEQUENCE DESCRIPTION: SEQ ID NO:7:
   Met Ser Arg His Leu Asn Val Ala Ile Val Gly Ala Thr Gly Ala Val
   1               5                   10                  15
   Gly Glu Thr Phe Leu Thr Val Leu Glu Glu Arg Asn Phe Pro Ile Lys
               20                  25                  30
   Ser Leu Tyr Pro Leu Ala Ser Ser Arg Ser Val Gly Lys Thr Val Thr
           35                  40                  45
   Phe Arg Asp Gln Glu Leu Asp Val Leu Asp Leu Ala Glu Phe Asp Phe
       50                  55                  60
   Ser Lys Val Asp Leu Ala Leu Phe Ser Ala Gly Gly Ala Val Ser Lys
   65                  70                  75                  80
   Glu Tyr Ala Pro Lys Ala Val Ala Ala Gly Cys Val Val Val Asp Asn
                   85                  90                  95
   Thr Ser Cys Phe Arg Tyr Glu Asp Asp Ile Pro Leu Val Val Pro Gly
               100                 105                 110
   Ser Glu Ser Ser Ser Asn Arg Asp Tyr Thr Lys Arg Gly Ile Ile Ala
           115                 120                 125
   Asn Pro Asn Cys Ser Thr Ile Gln Met Val Val Ala Leu Lys Pro Ile
       130                 135                 140
   Tyr Asp Ala Val Gly Ile Ser Arg Ile Asn Val Ala Thr Tyr Gln Ser
   145                 150                 155                 160
(2) INFORMATION FOR SEQ ID NO:8:
   (i) SEQUENCE CHARACTERISTICS:
      (A) LENGTH: 1054 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: cen3n.pk0067.a3
   (xi) SEQUENCE DESCRIPTION: SEQ ID NO:8:
   ATTTAACGGA AATGGGAAGA CACTCGAACA TCTTAAATTA GCTGCTGAGA GTGGAGTATT    60
   TGTAAATGTG GATAGCGAAT TTGATTTGGA GAATATTGTC AGAGCTGCAA GAGCTACTGG   120
   AAAGAAAGTG CCTGTTTTGC TTCGAATAAA TCCAGATGTG GATCCGCAGG TACATCCTTA   180
   TGTTGCCACG GGAAATAAAA CGTCTAAATT TGGGATCCGC AATGAGAAAT TGCAATGGTT    240
   TTTGGACTCT ATCAAGTCAT ACCCGAATGA AATCAAACTC GTTGGTGTTC ATTGCCATCT    300
   GGGATCTACT ATTACAAAGG TTGATATATT CAGAGATGCT GCAGTTCTTA TGCTGAATTA    360
   TGTCGATGAA ATTCGAGCAC AAGGTTTTAA GTTGGAGTAC CTGAATATCG GAGGTGGTTT    420
   GGGAATAGAT TACCATCATA CCGATGCAGT CTTACCTACA CCTATGGATC TCATCAACAC    480
   TGTGCGAGAA TTAGTTCTCT CTCAAGATCT CACTCTTATT ATTGAACCCG GAAGATCCTT    540
   GATTGCTAAT ACTTGCTGCT TCGTCAATAG AGTAACTGGT GTTAAATCTA ATGGTACAAA    600
   GAATTTCATT GTTGTTGATG GCAGCATGGC AGAACTCATC AGACCTAGTC TGTATGGAGC    660
   ATACCAGCAT ATCGAACTGG TCTCTCCCCC CACTCCTGGT GCTGAAGCAG CGACCTTCGA    720
   TATTGTTGGA CCAGTTTGTG AGTCTGCAGA TTTCCTTGGA AAAGATAGGG AACTTCCAAC    780
   ACCTGATGAG GGAGCTGGAC TGGTTGTTCA TGATGCAGGT GCCTACTGCA TGAGCATGGC    840
   TTCCACCTAC AACCTGAAGT TGAGGCCACC GGAATACTGG GTGGAAGCGG ACGGTTCGAT    900
   CGTTAAGATC AGGCATGGAG AGAAGCTTGA TGACTACATG AAGTTCTTTG ATGGTCTTCC    960
   TGCTTAGATG TTTATTATCT GCGACTGCTA CGGACGATGT TTTCTTGGGG ATAATTGGAT   1020
   TTTCTTTGTC AAAAAAAAAA AAAAAAAAAA AAAA                               1054
(2) INFORMATION FOR SEQ ID NO:9:
   (i) SEQUENCE CHARACTERISTICS:
      (A) LENGTH: 321 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: cen3n.pk0067.a3
   (xi) SEQUENCE DESCRIPTION: SEQ ID NO:9:
   Phe Asn Gly Asn Gly Lys Thr Leu Glu His Leu Lys Leu Ala Ala Glu
   1               5                   10                  15
   Ser Gly Val Phe Val Asn Val Asp Ser Glu Phe Asp Leu Glu Asn Ile
               20                  25                  30
   Val Arg Ala Ala Arg Ala Thr Gly Lys Lys Val Pro Val Leu Leu Arg
           35                  40                  45
   Ile Asn Pro Asp Val Asp Pro Gln Val His Pro Tyr Val Ala Thr Gly
       50                  55                  60
   Asn Lys Thr Ser Lys Phe Gly Ile Arg Asn Glu Lys Leu Gln Trp Phe
   65                  70                  75                  80
   Leu Asp Ser Ile Lys Ser Tyr Pro Asn Glu Ile Lys Leu Val Gly Val
                   85                  90                  95
   His Cys His Leu Gly Ser Thr Ile Thr Lys Val Asp Ile Phe Arg Asp
               100                 105                 110
   Ala Ala Val Leu Met Leu Asn Tyr Val Asp Glu Ile Arg Ala Gln Gly
           115                 120                 125
   Phe Lys Leu Glu Tyr Leu Asn Ile Gly Gly Gly Leu Gly Ile Asp Tyr
       130                 135                 140
   His His Thr Asp Ala Val Leu Pro Thr Pro Met Asp Leu Ile Asn Thr
   145                 150                 155                 160
   Val Arg Glu Leu Val Leu Ser Gln Asp Leu Thr Leu Ile Ile Glu Pro
                   165                 170                 175
   Gly Arg Ser Leu Ile Ala Asn Thr Cys Cys Phe Val Asn Arg Val Thr
               180                 185                 190
   Gly Val Lys Ser Asn Gly Thr Lys Asn Phe Ile Val Val Asp Gly Ser
           195                 200                 205
   Met Ala Glu Leu Ile Arg Pro Ser Leu Tyr Gly Ala Tyr Gln His Ile
       210                 215                 220
   Glu Leu Val Ser Pro Pro Thr Pro Gly Ala Glu Ala Ala Thr Phe Asp
   225                 230                 235                 240
   Ile Val Gly Pro Val Cys Glu Ser Ala Asp Phe Leu Gly Lys Asp Arg
                   245                 250                 255
   Glu Leu Pro Thr Pro Asp Glu Gly Ala Gly Leu Val Val His Asp Ala
               260                 265                 270
   Gly Ala Tyr Cys Met Ser Met Ala Ser Thr Tyr Asn Leu Lys Leu Arg
           275                 280                 285
   Pro Pro Glu Tyr Trp Val Glu Ala Asp Gly Ser Ile Val Lys Ile Arg
       290                 295                 300
   His Gly Glu Lys Leu Asp Asp Tyr Met Lys Phe Phe Asp Gly Leu Pro
   305                 310                 315                 320
   Ala
(2) INFORMATION FOR SEQ ID NO:10:
   (i) SEQUENCE CHARACTERISTICS:
      (A) LENGTH: 1813 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: crln.pk0103.d8
   (xi) SEQUENCE DESCRIPTION: SEQ ID NO:10:
   CGCTTCCTGG AAGGCTGGAA CAGAAAGAAC CCTAAACCCT AGCAATGGCG GCGGCGAACC    60
   TGCTGTCGCG CTCCCTTCTC CCCACCCCAA ACACTATCCG AACGAGCCAC CCCACCCCGC   120
   GGAGCCCAGC CGTCGTCTCC TTCCCCCGCC GCCGTGCCCG CCTGTCCGTG TGCGCCTCCG   180
   TCTCCATGGC CTCCCCGTCC CCACCGCCAC AGCCCGCGGC GGCCGGCGTG CCGAAGCACT   240
   GCTTCCGGCG CGGCGCCGAC GGCTACCTGT ACTGCGAGGG AGTGAGGGTG GAAGACGCGA   300
   TGGCGGCTGC CGAGCGCAGC CCCTTCTATC TCTACAGCAA GCTTCAGATC CTCCGCAACT     360
   TCGCCGCTTA CCGCGACGCT CTCCAGGGGC TCCGCTCCAT CGTCGGGTAT GCCGTGAAGG     420
   CCAACAATAA CCTCCCCGTG CTACGCGTCC TGCGTGAGCT TGGCTGCGGC GCCGTCCTCG     480
   TCAGCGGCAA CGAGCTCCGA CTCGCCCTCC AGGCGGGATT CGACCCCGCC AGGTGTATAT     540
   TTAACGGAAA TGGGAAGACA CTCGAAGATC TTAAATTGGC TGCTGAGAGT GGAGTATTTG     600
   TAAATGTGGA TAGTGAATTT GATTTAGAGA ATATTGTCAG AGCTGCAAGA GCTACTGGAA     660
   AGAAAGTGCC TGTTTTACTT AGAATAAATC CAGATGTGGA TCCACAGGTA CATCCATATG     720
   TTGCCACGGG AAATAAAACA TCCAAATTCG GGATCCGCAA TGAGAAATTG CAATGGTTTT     780
   TGAACTCTAT CAAGTCATAC TCGAATGAAA TCAAACTCGT TGGTGTTCAT TGCCATCTGG     840
   GATCTACTAT TACAAAGGTT GATATATTCA GAGATGCTGC AGTGCTTATG GTGAATTATG     900
   TCGATGAAAT TCGAGCACAA GGTTTTAAGT TGGAGTACCT GAATATTGGA GGTGGTTTGG     960
   GAATAGATTA CCATCATACC GATGCAGTCT TACCTACACC TATGGATCTC ATCAACACTG    1020
   TACGAGAATT AGTTCTCTCT CAAGATCTTA CTCTTATTAT TGAACCTGGA AGATCCTTGA    1080
   TTGCTAATAC TTGCTGCTTC GTCAATAGAG TAACTGGTGT TAAATCTAAT GGTACAAAGA    1140
   ATTTCATTGT TGTTGATGGC AGCATGGCAG AACTCATCAG ACCTAGCCTG TATGGAGCAT    1200
   ATCAGCATAT CGAATTGGTC TCTCCCCCCA CTCCTGGTGC TGAAGTAGCG ACCTTCGATA    1260
   TTGTTGGGCC AGTTTGTGAG TCTGCAGATT TCCTTGGAAA AGATAGGGAA CTTCCAACAC    1320
   CTGATGAGGG AGCTGGACTG GTTGTTCATG ATGCAGGTGC CTACTGCATG AGCATGGCTT    1380
   CCACCTACAA CCTGAAGTTG AGGCCGCCAG AGTACTGGGT TGAAGAGGAT GGTTCGATTG    1440
   TTAAGATCAG GCATGAAGAG AAGCTCGATG ACTACATGAA GTTCTTTGAT GGTCTTCCTG    1500
   CTTAGATGTT TATTTGTGAC TGCTAGGGGC GATGTTTTCT TGGAGATAAT TGAATTTTTC    1560
   TTTGTCAAGC TCATTTTGCT TTCTTGTGGT TGTTATGGAA TGTTACTGGA TACTGGATAG    1620
   TTAGTTCGGC CTGTAGGCGT ATCCTCCTGA ACTTACCTCT CATTGCTGTT AGTTTTGGCA    1680
   CCAAGTTTGT TCCCAATTGC TATTTACGGA AGTTATTGCA TAAAGGGCTG TTTGGTTGTA    1740
   ATCTTCCCGT AAGAATAAGA TGCATGTTTT TGAGTTAAAA AAGGGGGGGC CCGGTACCCA    1800
   ATTCGCCCTA TAG                                                       1813
(2) INFORMATION FOR SEQ ID NO:11:
   (i) SEQUENCE CHARACTERISTICS:
      (A) LENGTH: 486 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: cr1n.pk0103.d8
   (xi) SEQUENCE DESCRIPTION: SEQ ID NO:11:
   Met Ala Ala Ala Asn Leu Leu Ser Arg Ser Leu Leu Pro Thr Pro Asn
   1 5 10 15
   Thr Ile Arg Thr Ser His Pro Thr Pro Arg Ser Pro Ala Val Val Ser
   20 25 30
   Phe Pro Arg Arg Arg Ala Arg Leu Ser Val Cys Ala Ser Val Ser Met
   35 40 45
   Ala Ser Pro Ser Pro Pro Pro Gln Pro Ala Ala Ala Gly Val Pro Lys
   50 55 60
   His Cys Phe Arg Arg Gly Ala Asp Gly Tyr Leu Tyr Cys Glu Gly Val
   65 70 75 80
   Arg Val Glu Asp Ala Met Ala Ala Ala Glu Arg Ser Pro Phe Tyr Leu
   85 90 95
   Tyr Ser Lys Leu Gln Ile Leu Arg Asn Phe Ala Ala Tyr Arg Asp Ala
   100 105 110
   Leu Gln Gly Leu Arg Ser Ile Val Gly Tyr Ala Val Lys Ala Asn Asn
   115 120 125
   Asn Leu Pro Val Leu Arg Val Leu Arg Glu Leu Gly Cys Gly Ala Val
   130 135 140
   Leu Val Ser Gly Asn Glu Leu Arg Leu Ala Leu Gln Ala Gly Phe Asp
   145 150 155 160
   Pro Ala Arg Cys Ile Phe Asn Gly Asn Gly Lys Thr Leu Glu Asp Leu
   165 170 175
   Lys Leu Ala Ala Glu Ser Gly Val Phe Val Asn Val Asp Ser Glu Phe
   180 185 190
   Asp Leu Glu Asn Ile Val Arg Ala Ala Arg Ala Thr Gly Lys Lys Val
   195 200 205
   Pro Val Leu Leu Arg Ile Asn Pro Asp Val Asp Pro Gln Val His Pro
   210 215 220
   Tyr Val Ala Thr Gly Asn Lys Thr Ser Lys Phe Gly Ile Arg Asn Glu
   225 230 235 240
   Lys Leu Gln Trp Phe Leu Asn Ser Ile Lys Ser Tyr Ser Asn Glu Ile
   245 250 255
   Lys Leu Val Gly Val His Cys His Leu Gly Ser Thr Ile Thr Lys Val
   260 265 270
   Asp Ile Phe Arg Asp Ala Ala Val Leu Met Val Asn Tyr Val Asp Glu
   275 280 285
   Ile Arg Ala Gln Gly Phe Lys Leu Glu Tyr Leu Asn Ile Gly Gly Gly
   290 295 300
   Leu Gly Ile Asp Tyr His His Thr Asp Ala Val Leu Pro Thr Pro Met
   305 310 315 320
   Asp Leu Ile Asn Thr Val Arg Glu Leu Val Leu Ser Gln Asp Leu Thr
   325 330 335
   Leu Ile Ile Glu Pro Gly Arg Ser Leu Ile Ala Asn Thr Cys Cys Phe
               340                 345                 350
   Val Asn Arg Val Thr Gly Val Lys Ser Asn Gly Thr Lys Asn Phe Ile
           355                 360                 365
   Val Val Asp Gly Ser Met Ala Glu Leu Ile Arg Pro Ser Leu Tyr Gly
       370                 375                 380
   Ala Tyr Gln His Ile Glu Leu Val Ser Pro Pro Thr Pro Gly Ala Glu
   385                 390                 395                 400
   Val Ala Thr Phe Asp Ile Val Gly Pro Val Cys Glu Ser Ala Asp Phe
                   405                 410                 415
   Leu Gly Lys Asp Arg Glu Leu Pro Thr Pro Asp Glu Gly Ala Gly Leu
               420                 425                 430
   Val Val His Asp Ala Gly Ala Tyr Cys Met Ser Met Ala Ser Thr Tyr
           435                 440                 445
   Asn Leu Lys Leu Arg Pro Pro Glu Tyr Trp Val Glu Glu Asp Gly Ser
       450                 455                 460
   Ile Val Lys Ile Arg His Glu Glu Lys Leu Asp Asp Tyr Met Lys Phe
   465                 470                 475                 480
   Phe Asp Gly Leu Pro Ala
                   485
(2) INFORMATION FOR SEQ ID NO:12:
   (i) SEQUENCE CHARACTERISTICS:
      (A) LENGTH: 1116 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
(3) CLONE: r10n.pk0013.b9
   (xi) SEQUENCE DESCRIPTION: SEQ ID NO:12:
   CTTACACGGA GTGTTTGTAA ACATAGACAG TGAATTTGAT TTGGAGAATA TTGTCACTGC 60
   TGCGAGAGTT GCTGGGAAGA AAGTCCCTGT TTTGCTCAGG ATAAACCCAG ATGTGGATCC 120
   ACAGGTCCAT CCTTATGTTG CGACTGGAAA CAAAACCTCC AAATTTGGTA TCCGTAATGA 180
   GAAACTACAA TGGTTCTTAG ACTCTATCAA GTCATACTCA AATGATATCA CACTGGTGGG 240
   TGTTCATTGT CATCTGGGAT CTACCATTAC AAAGGTCGAT ATATTTAGAG ATGCGGCAGG 300
   TCTTATGGTG AATTATGTTG ATGAAATTCG AGCACAAGGT TTTGAACTGG AATATCTCAA 360
   TATTGGCGGT GGCCTGGGCA TAGWTTATCA CCACACGGAT GCAGTCTTGC CTACACCTAT 420
   GGGACCTCAT CAACACTGTG CCGAAGAATT AGTTCTGTCA CGAGATCTTA CACTCATCAT 480
   TGAACCTGGG AGATCCCTCA TAGCTAACAC TTGCTGCTTC GTCAATAGGG TCACTGGTGT 540
   TAAATCTAAT GGTACAAAGA ATTTCATTGT AGTTGATGGC AGCATGGCAG AGCTTATCAG 600
   ACCAAGTCTA TATGGAGCAT ACCAGCATAT CGAACTGGTT TCTCCTTCCC CAGATGCAGA 660
   AGTAGCAACA TTCGATATTG TTGGACCAGT TTGTGAATCT GCAGATTTCC TTGGCAAAGA 720
   CAGGGAACTT CCAACACCTG ATAAGGGAGC TGGTTTGGTG GTTCATGACG CAGGAGCCTA 780
   CTGCATGAGC ATGGCTTCAA CCTACAACTT GAAGTTGCGA CCACCTGAAT ATTGGGTAGA 840
   AGATGATGGG TCCATTGCTA AGATTCGGCG TGGAGAGTCA TTTGATGACT ACATGAAGTT 900
   CTTTGATAAT CTCTCTGCCT AACTCGTTTT CCTGCAATTG TAATAAGATT TTTCTCTTGT 960
   TATGTGTGGC TGTATCAGGA TTCGGATTGA TAGCGCAGTA CAGTTTGCTG TAGAATCGGT 1020
   ATTTTTTTTT ATTGTACTGT GATGTCGGTA CCTTATTTTA TCCAAAGATT TTTGGCAAAT 1080
   TTTGCTACAG GACACTTAAA AAAAAAAAAA AAAAAA 1116
(2) INFORMATION FOR SEQ ID NO:13:
   (i) SEQUENCE CHARACTERISTICS:
      (A) LENGTH: 306 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: r10n,pk0013.b9
   (xi) SEQUENCE DESCRIPTION: SEQ ID NO:13:
   Leu His Gly Val Phe Val Asn Ile Asp Ser Glu Phe Asp Leu Glu Asn
   1               5                   10                  15
   Ile Val Thr Ala Ala Arg Val Ala Gly Lys Lys Val Pro Val Leu Leu
               20                  25                  30
   Arg Ile Asn Pro Asp Val Asp Pro Gln Val His Pro Tyr Val Ala Thr
           35                  40                  45
   Gly Asn Lys Thr Ser Lys Phe Gly Ile Arg Asn Glu Lys Leu Gln Trp
       50                  55                  60
   Phe Leu Asp Ser Ile Lys Ser Tyr Ser Asn Asp Ile Thr Leu Val Gly
   65                  70                  75                  80
   Val His Cys His Leu Gly Ser Thr Ile Thr Lys Val Asp Ile Phe Arg
                   85                  90                  95
   Asp Ala Ala Gly Leu Met Val Asn Tyr Val Asp Glu Ile Arg Ala Gln
               100                 105                 110
   Gly Phe Glu Leu Glu Tyr Leu Asn Ile Gly Gly Gly Leu Gly Ile Xaa
           115                 120                 125
   Tyr His His Thr Asp Ala Val Leu Pro Thr Pro Met Gly Pro His Gln
       130                 135                 140
   His Cys Ala Glu Glu Leu Val Leu Ser Arg Asp Leu Thr Leu Ile Ile
   145                 150                 155                 160
   Glu Pro Gly Arg Ser Leu Ile Ala Asn Thr Cys Cys Phe Val Asn Arg
                   165                 170                 175
   Val Thr Gly Val Lys Ser Asn Gly Thr Lys Asn Phe Ile Val Val Asp
               180                 185                 190
   Gly Ser Met Ala Glu Leu Ile Arg Pro Ser Leu Tyr Gly Ala Tyr Gln
           195                 200                 205
   His Ile Glu Leu Val Ser Pro Ser Pro Asp Ala Glu Val Ala Thr Phe
       210                 215                 220
   Asp Ile Val Gly Pro Val Cys Glu Ser Ala Asp Phe Leu Gly Lys Asp
   225                 230                 235                 240
   Arg Glu Leu Pro Thr Pro Asp Lys Gly Ala Gly Leu Val Val His Asp
                   245                 250                 255
   Ala Gly Ala Tyr Cys Met Ser Met Ala Ser Thr Tyr Asn Leu Lys Leu
               260                 265                 270
   Arg Pro Pro Glu Tyr Trp Val Glu Asp Asp Gly Ser Ile Ala Lys Ile
           275                 280                 285
   Arg Arg Gly Glu Ser Phe Asp Asp Tyr Met Lys Phe Phe Asp Asn Leu
       290                 295                 300
   Ser Ala
   305
(2) INFORMATION FOR SEQ ID NO:14:
   (i) SEQUENCE CHARACTERISTICS:
      (A) LENGTH: 968 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: sr1.pk0132.c1
   (xi) SEQUENCE DESCRIPTION: SEQ ID NO:14:
   GTTGCCACTG GGAATAAGAA CTCTAAATTT GGCATTAGAA ATGAGAAGCT GCAGTGCTTT    60
   TTAGATGCAG TGAAGGAACA TCCTAATGAG CTCAAACTTG TAGGGGCCCA CTGCCATCTT   120
   GGTTCAACAA TTACCAAGGT TGACATTTTC AGGGATGCAG CCACCATTAT GATCAACTAC   180
   ATTGACCAAA TCCGAGATCA GGGTTTTGAA GTTGATTACT TAAATATTGG TGGAGGACTT   240
   GGGATAGATT ATTATCATTC TGGTGCCATC CTTCCTACAC CTAGAGATCT CATTGACACT   300
   GTACGAGATC TTGTTATTTC ACGTGGTCTT AATCTCATCA TTGAACCAGG AAGATCACTC   360
   ATTGCAAACA CGTGTTGCTT AGTTAACCGG GTGACAGGTG TTAAAACTAA TGGATCTAAA   420
   AACTTCATTG TAATTGATGG AAGTATGGCT GAACTTATCC GCCCTAGTCT TTATGATGCT   480
   TACCAGCATA TAGAGCTGGT TTCCCCTGCC CCGTCAAATG CTGAAACAGA AACTTTTGAT   540
   GTGGTTGGCC CTGTCTGTGA GTCTGCAGAT TTCTTAGGAA AAGGAAGAGA ACTTCCTACT   600
   CCAGCCAAGG GTACTGGTTT GGTTGTTCAT GATGCTGGTG CTTATTGCAT GAGCATGGCA   660
   TCAACCTACA ATCTAAAGAT GCGGCCTCCT GAGTATTGGG TTGAAGATGA TGGATCAGTG   720
   AGCAAAATAA GACATGGAGA GACTTTTGAA GACCACATTC GGTTTTTTGA GGGGCTTTGA   780
   GCTAATAATT TATCTTGTAG GAAAGAAGGC TGGAGAATTG TTATGTACTT GGAGTTTGAA   840
   TCTTTCCTCG TCAATGAATG CATGACTCTT GTAGTTCTGT TTCTTCCGTT CTAATTGAAT   900
   GTTGACTCCC ATGACAGGAA CAGAGAATAA AGTTGATTTC AGTTAGATTT AAAAAAAAAA   960
   AAAAAAAA                                                            968
(2) INFORMATION FOR SEQ ID NO:15:
   (i) SEQUENCE CHARACTERISTICS:
      (A) LENGTH: 259 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: srl.pk0132.c1
   (xi SEQUENCE DESCRIPTION: SEQ ID NO:15:
(2) INFORMATION FOR SEQ ID NO:16:
   (i) SEQUENCE CHARACTERISTICS:
      (A) LENGTH: 676 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: wlkl.pk0012.c2
   (xi) SEQUENCE DESCRIPTION: SEQ ID NO:16:
   TTTGAGTTGG AGTACCTGAA TATTGGAGGT GGTTTGGGGA TAGACTACCA CCACACTGGT    60
   GCAGTCTTGC CTACACCTAT GGATCTTATC AACACTGTCC GGGAATTGGT CCTCTCACGG   120
   GATCTTACTC TCATTATTGA ACCTGGAAGA TCCCTGATCG CCAATACTTG CTGCTTCGTC   180
   AATAAGGTCA CTGGTGTAAA ATCGAATGGC ACGAAGAATT TCATTGTAGT TGATGGCAGC   240
   ATGGCCGAGC TCATCAGGCC TAGTCTATAT GGAGCATATC AGCATATAGA ACTAGTTCTC   300
   CCTCTCCAAG GTGCAGAAGT AGCAACCTTC CGATATTGTT GGGGCCAGTC TGCGAATCTG   360
   CAGATTCCTT GGNAAAGACA AGGAGTTCCA ACACCTGACA AGGGANCTGG TTTGGGTGTC   420
   CACGACGCAN GANCTACTGC ATGAGCATGG CTTCNACCTA CAACCTGAAG ATGAGGCAAC   480
   CGAGTATTGG GTANAGGACA TGGNCCATGT AAGATAAGCA CGGGGAAACA TTGACGACAC   540
   ATGAGTCTTG ATNGCTCCGC CAGGCCTTTA CTGGTTGGNA ACNAGCTTCA TTGTNNCCAC   600
   CGTGGAATCT GGGAACATCN TGTTGTAGTG GCACCACANA GGGNTTTTGN GACAATCACA   660
   NTAGATGAGA TTNTGG                                                   676
(2) INFORMATION: FOR SEQ ID NO:17:
   (i) SEQUENCE CHARACTERISTICS:
      (A) LENGTH: 73 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: wlkl.pk0012.c2
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 544 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: sdp3c.pk001.015
   (xi) SEQUENCE DESCRIPTION: SEQ ID NO:18:
   TTGCAACACA CATTGTCTTG TCGGCAAAAT CTTCCACCAA CAACACACAG CCATGGCAGG    60
   CTCAAACATT CTTTCTCACT CTCCTTCCCT TCCCAAAACC TACAGCCACT CCTTAAACCA   120
   AAACGCGTTA TCCCAAAAGC TTTTTTTTCT GCCCCTCAAA TTCAAAGCCA CCACAAAACC   180
   ACGTGCTCTC AGAGCGGTTC TCTCGCAGAA CGCTGTCAAA ACCTCGGTGG AGGACACAAA   240
   GAACGCTCAT TTTCAGCACT GTTTCACCAA ATCCGAAGAT GGGTATCTGT ACTGTGAGGG   300
   CCTCAAGGTG CATGACATCA TGGAATCTGT TGAGAGAAGA CCTTTCTATT TGTACAGCAA   360
   GCCCCAGATA ACTAGGAATG TTGAAGCCTA CAAGGATGCA TTGGAAGGGT TGAACTCCAT   420
   AATTGGTTAT GCCATTAAGG CCAATAATAA CTTGAAGATT TTGGNACATT TGAGGCACTT   480
   GGGTTGTGGT GCTGTGCTTG TTAGTGGGAA TGAGCTGAAG TTGNTCTTCG AGCTGGNTTT   540
   GTTC                                                                544
(2) INFORMATION FOR SEQ ID NO:19:
   (i) SEQUENCE CHARACTERISTICS:
      (A) LENGTH: 62 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: sdp3c.pk001.o15
   (xi) SEQUENCE DESCRIPTION: SEQ ID NO:19:
   Arg Arg Pro Phe Tyr Leu Tyr Ser Lys Pro Gln Ile Thr Arg Asn Val
   1               5                   10                  15
   Glu Ala Tyr Lys Asp Ala Leu Glu Gly Leu Asn Ser Ile Ile Gly Tyr
               20                  25                  30
   Ala Ile Lys Ala Asn Asn Asn Leu Lys Ile Leu Xaa His Leu Arg His
           35                  40                  45
   Leu Gly Cys Gly Ala Val Leu Val Ser Gly Asn Glu Leu Lys
       50                  55                  60
(2) INFORMATION FOR SEQ ID NO:20:
   (i) SEQUENCE CHARACTERISTICS:
      (A) LENGTH: 371 amino acids
      (B) TYPE: amino acid
      (C) STRANDEDNESS: not relevant
      (D) TOPOLOGY: linear
   (ii) MOLECULE TYPE: peptide
   (vi) ORIGINAL SOURCE:
      (A) ORGANISM: Pseudomonas aeruginosa
   (xi) SEQUENCE DESCRIPTION: SEQ ID NO:20:
   Met Lys Arg Val Gly Leu Ile Gly Trp Arg Gly Met Val Gly Ser Val
   1               5                   10                  15
   Leu Ile Gln Arg Met Leu Glu Glu Arg Asp Phe Asp Leu Ile Glu Pro
               20                  25                  30
   Val Phe Phe Thr Thr Ser Asn Val Gly Ala Gln Ala Pro Glu Val Asp
           35                  40                  45
   Lys Asp Ile Ala Pro Leu Lys Asp Ala Tyr Ser Ile Asp Glu Leu Lys
       50                  55                  60
   Thr Leu Asp Val Ile Leu Thr Cys Gln Gly Gly Asp Tyr Thr Ser Glu
   65                  70                  75                  80
   Val Phe Pro Lys Leu Arg Glu Ala Gly Trp Gln Gly Tyr Trp Ile Asp
                   85                  90                  95
   Ala Ala Ser Ser Leu Arg Met Glu Asp Asp Ala Val Ile Val Leu Asp
               100                 105                 110
   Pro Val Asn Arg Lys Val Ile Asp Gln Ala Leu Asp Ala Gly Thr Arg
           115                 120                 125
   Asn Tyr Ile Gly Gly Asn Cys Thr Val Ser Leu Met Leu Met Ala Leu
       130                 135                 140
   Gly Gly Leu Phe Asp Ala Gly Leu Val Glu Trp Met Ser Ala Met Thr
   145                 150                 155                 160
   Tyr Gln Ala Ala Ser Gly Ala Gly Ala Gln Asn Met Arg Asp Leu Leu
                   165                 170                 175
   Lys Gln Met Gly Ala Ala His Ala Ser Val Ala Asp Asp Leu Ala Asn
               180                 185                 190
   Pro Ala Ser Ala Ile Leu Asp Ile Asp Arg Lys Val Ala Glu Thr Leu
           195                 200                 205
   Arg Ser Glu Ala Phe Pro Thr Glu His Phe Gly Ala Pro Leu Gly Gly
       210                 215                 220
   Ser Leu Ile Pro Trp Ile Asp Lys Glu Leu Ser Gln Arg Arg Gln Ser
   225                 230                 235                 240
   Arg Glu Glu Trp Lys Ala Gln Ala Glu Thr Asn Lys Ile Leu Ala Arg
                   245                 250                 255
   Phe Lys Asn Pro Ile Pro Val Asp Gly Ile Cys Val Arg Val Gly Ala
               260                 265                 270
   Met Arg Cys His Ser Gln Ala Leu Thr Ile Lys Leu Asn Lys Asp Val
           275                 280                 285
   Pro Leu Thr Asp Ile Glu Gly Leu Ile Arg Gln His Asn Pro Trp Val
       290                 295                 300
   Lys Leu Val Pro Asn His Arg Glu Val Ser Val Arg Glu Leu Thr Pro
   305                 310                 315                 320
   Ala Ala Val Thr Gly Thr Leu Ser Val Pro Val Gly Arg Leu Arg Lys
                   325                 330                 335
   Leu Asn Met Val Ser Gln Tyr Leu Gly Ala Phe Thr Val Gly Asp Gln
               340                 345                 350
   Leu Leu Trp Gly Ala Ala Glu Pro Leu Arg Arg Met Leu Arg Ile Leu
           355                 360                 365
   Leu Glu Arg
       370
(2) INFORMATION FOR SEQ ID NO:21:
   (i) SEQUENCE CHARACTERISTICS:
      (A) LENGTH: 788 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: crln.pk0009.g4
   (xi) SEQUENCE DESCRIPTION: SEQ ID NO:21:
   CGACAACATC GCCCCCGCCA TCCTCGGCGG CTTCGTCCTC GTCCGCAGCT ACGACCCCTT    60
   TCACCTCGTC CCGCTTTCCT TCCCGCCAGC GCTCCGCCTC CACTTCGTCC TGGTCACCCC   120
   CGACTTCGAG GCGCCCACGA GCAAGATGCG CGCCGCGCTG CCCAGGCAGG TCGACGTCCA   180
   GCAGCACGTG CGCAACTCCA GCCAGGCAGC GGCGCTCGTG GCGGCGGTGC TGCAGGGGGA   240
   CGCGGGCCTC ATCGGCTCCG CGATGTCGTC CGACGGCATC GTGGAGCCCA CCAGGGCACC   300
   CCTCATACCT GGCATGGCGG CCGTAAAGGC GGCGGCCCTG CAAGCTGGAG CGCTGGGCTG   360
   CACAATTAGC GGCGCGGGCC CCACAGTGGT GGCCGTCATC CAAGGGGAGG AAAGGGGGGA   420
   GGAGGTTGCC CGCAAGATGG TGGACGCGTT CTGGAGCGCA GGCAAGCTCA AGGCGACAGC   480
   AACCGTCGCG CAGCTCGATA CCCTTGGTGC CAGGGTCATC GCCACGTCAT CCTTGAACTA   540
   GCAAAAGATT CGGAAAGTGG TACTGCAATT GTATCACCAA ACAAGGAAGA ATGAAGGGGA   600
   ACCCCATGGA TTTGTATGTT TTCTCTTCTT TCTTGCATCT TTAGGTGGTT AATTGGCTTT   660
   GGAATAAATG AGATGGAGGA CATCGCTAGA ACAATTCTGT TCCGTGGGCT GTAATTTCAA   720
   TTTGGGCTGG TTTCTTTATC ATGCCATGGA TAATTATGAA TAAATTTGAG GTAGTTTGTT   780
   AAAAAAAA                                                             788
(2) INFORMATION FOR SEQ ID NO:22:
   (i) SEQUENCE CHARACTERISTICS:
      (A) LENGTH: 179 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: crln.pk0009.g4
   (xi) SEQUENCE DESCRIPTION: SEQ ID NO:22:
   Asp Asn Ile Ala Pro Ala Ile Leu Gly Gly Phe Val Leu Val Arg Ser
   1               5                   10                  15
   Tyr Asp Pro Phe His Leu Val Pro Leu Ser Phe Pro Pro Ala Leu Arg
               20                  25                  30
   Leu His Phe Val Leu Val Thr Pro Asp Phe Glu Ala Pro Thr Ser Lys
           35                  40                  45
   Met Arg Ala Ala Leu Pro Arg Gln Val Asp Val Gln Gln His Val Arg
       50                  55                  60
   Asn Ser Ser Gln Ala Ala Ala Leu Val Ala Ala Val Leu Gln Gly Asp
   65                  70                  75                  80
   Ala Gly Leu Ile Gly Ser Ala Met Ser Ser Asp Gly Ile Val Glu Pro
                   85                  90                  95
   Thr Arg Ala Pro Leu Ile Pro Gly Met Ala Ala Val Lys Ala Ala Ala
               100                 105                 110
   Leu Gln Ala Gly Ala Leu Gly Cys Thr Ile Ser Gly Ala Gly Pro Thr
           115                 120                 125
   Val Val Ala Val Ile Gln Gly Glu Glu Arg Gly Glu Glu Val Ala Arg
       130                 135                 140
   Lys Met Val Asp Ala Phe Trp Ser Ala Gly Lys Leu Lys Ala Thr Ala
   145                 150                 155                 160
   Thr Val Ala Gln Leu Asp Thr Leu Gly Ala Arg Val Ile Ala Thr Ser
                   165                 170                 175
   Ser Leu Asn
(2) INFORMATION FOR SEQ ID NO:23:
   (i) SEQUENCE CHARACTERISTICS:
      (A) LENGTH: 601 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: rcal1.pk005.k3
   (xi) SEQUENCE DESCRIPTION: SEQ ID NO:23:
   GTCGCCGCCA TCGCTGCCCT TCGCGCCCTC GATGTCAAGT CCCACGCCGT CTCCATCCAC    60
   CTCACCAAGG GCCTCCCCCT CGGCTCCGGC CTCGGCTCCT CCGCCGCCTC CGCCGCCGCC   120
   GCTGCCAAGG CCGTTGACGC CCTCTTCGGC TCCCTCCTAC ACCAAGATGA CCTCGTCCTC   180
   GCGGGCCTCG AGTCCGAGAA AGCCGTCAGT GGCTTCCACG CCGACAACAT CGCCCCGGCC   240
   ATCCTCGGCG GCTTCGTCCT CGTCCGCAGC TACGACCCCT TCCACCTCAT CCCGCTCTCC   300
   TCCCCACCTG CCCTCCGCCT CCACTTCGTC CTCGTCACGC CCGACTTCGA GGCGCCCACC   360
   AAGCAAGATG CGTGCCGCGC TGCCCAAACA GGTGGCCGTC CACCAAGCAC GTCCGCAACT   420
   CCAGCCAAGC GGNCGCGCTT GTCGCCGCTG TGCTGCAAGG GGACGCCACC CTCATCGGCT   480
   CCGCAATGTC CTCCGACGGC ATCGTGGAGC CAACAAGGCG CCGCTGATTC TGGATGGCTG   540
   CGGTCAAAGG CGCCGGCTTG GAACTGGGGG AATTGGCTGC ACATCAGTGG AGAAGGCAAN   600
   T                                                                   601
(2) INFORMATION FOR SEQ ID NO:24:
   (i) SEQUENCE CHARACTERISTICS:
      (A) LENGTH: 82 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: rcalc.pk005.k3
   (xi) SEQUENCE DESCRIPTION: SEQ ID NO:24:
   Val Ser Ile His Leu Thr Lys Gly Leu Pro Leu Gly Ser Gly Leu Gly
   1               5                   10                  15
   Ser Ser Ala Ala Ser Ala Ala Ala Ala Ala Lys Ala Val Asp Ala Leu
               20                  25                  30
   Phe Gly Ser Leu Leu His Gln Asp Asp Leu Val Leu Ala Gly Leu Glu
           35                  40                  45
   Ser Glu Lys Ala Val Ser Gly Xaa Xaa His Ala Asp Asn Ile Ala Pro
       50                  55                  60
   Ala Ile Leu Gly Gly Phe Val Leu Val Arg Ser Tyr Asp Pro Phe His
   65                  70                  75                  80
   Leu Ile
(2) INFORMATION FOR SEQ ID NO:25:
   (i) SEQUENCE CHARACTERISTICS:
      (A) LENGTH: 1543 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: ses8w.pk0020.b5
   (xi) SEQUENCE DESCRIPTION: SEQ ID NO:25:
   GAAGAGAGAC AAACCAGCAA GAGTGGAGAT GGCGACGTCG ACGTGCTTCC TGTGTCCGTC   60
   TACGGCGAGT TTGAAAGGCA GGGCCAGATT CAGAATCAGA ATCAGATGCA GCAGCAGCGT  120
   GTCGGTCAAT ATTCGAAGGG AGCCCGAACC TGTAACGACG CTGGTGAAAG CGTTTGCTCC  180
   CGCCACGGTG GCGAATCTAG GTCCAGGCTT CGACTTCCTA GGCTGCGCCG TGGACGGACT  240
   CGGAGACATT GTGTCGGTGA AGGTTGACCC ACAGGTTCAC CCTGGCGAGA TATGCATATC  300
   CGACATCAGC GGCCACGCCC CAAACAAGCT CAGCAAAAAC CCTCTCTGGA ACTGCGCCGG  360
   CATCGCCGCC ATTGAAGTCA TGAAAATGCT CTCCATTCGA TCCGTCGGCC TCTCCCTCTC  420
   CCTGGAGAAG GGCCTGCCTT TGGGAAGCGG TCTGGGATCC AGCGCCGCCA GCGCCGCCGC  480
   GGCCGCCGTG GCGGTGAACG AGCTGTTTGG GAAGAAATTA AGCGTGGAGG AGCTGGTTCT  540
   GGCATCACTG AAATCGGAAG AGAAGGTGTC GGGGTATCAC GCGGACAACG TGGCGCCATC  600
   GATAATGGGG GGTTTTGTGC TGATCGGGAG CTACTCGCCG CTGGAGTTGA TGCCGTTGAA  660
   GTTTCCGGCA GAGAAGGAGC TGTATTTCGT GCTGGTGACG CCTGAGTTCG AGGCCCCGAC  720
   GAAGAAGATG CGGGCAGCGC TGCCTACGGA GATCGGGATG CCGCACCACG TGTGGAACTG  780
   CAGCCAGGCA GGTGCTCTGG TGGCGTCGGT GCTGCAGGGC GACGTGGTTG GGTTGCGGAA  840
   GGCATTGTCC TCTGACAAGA TCGTTGAGCC AAGGCGTGCC CCCTTGATTC CTGGCATGGA  900
   GGCTGTCAAG AGGGCTGCCA TTCAGGCCGG TGCTTTTGGC TGTACCATCA GCGGCGCCGG  960
   CCCTACCGCC GTCGCCGTCA TTGACGACGA GCAAACTGGA CACCTCATTG CCAAACACAT 1020
   GATTGACGCT TTTCTCCATG TTGGCAATTT GAAGGCTTCT GCAAATGTCA AGCAGCTTGA 1080
   TCGCCTTGGT GCTAGACGCA TTCCAAATTG AACCTTCTCT TCTCTATCTC TATGAGAGGC 1140
   TTGTAGATTT CAAGAACCGG ATTTCTTCCA ACTTGCTCGT AACACTCTAA GTGCTGACCG 1200
   GTCACATGTA TTTGAAATTT GATCTGATCA ATGAAGCAGC ATTCTAGTGT GGAGGTCTGA 1260
   ATAACAAGAG AAACATTAAA CCCAAGCTGG GAGCTCTGTT TGGGTGGTGG AAATTTAAAT 1320
   AGATGAATAA TTATGAAAGA CCTAGATCAG GTCAGTGTTA TGGTGAACTC TGAAGCATGT 1380
   TTTAGATTTT CTTTGCTTTG TTTTTATCAT ATTTTTATCT TGCTACTTGA GTTGACAAAG 1440
   CTCAAAAAGA AGTCATTTTT AGTATTTTCT TGTTTCATTA TGCTAGTTAA TCTTAGCTTT 1500
   TGAATAGCAT GTATTGTTCC TTAAAAAAAA AAAAAAAAAA AAA                   1543
(2) INFORMATION FOR SEQ ID NO:26:
   (i) SEQUENCE CHARACTERISTICS:
      (A) LENGTH: 483 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: ses8w.pk0020.b5
   (xi) SEQUENCE DESCRIPTION: SEQ ID NO:26:
   Met Ala Thr Ser Thr Cys Phe Leu Cys Pro Ser Thr Ala Ser Leu Lys
   1               5                   10                  15
   Gly Arg Ala Arg Phe Arg Ile Arg Ile Arg Cys Ser Ser Ser Val Ser
               20                  25                  30
   Val Asn Ile Arg Arg Glu Pro Glu Pro Val Thr Thr Leu Val Lys Ala
           35                  40                  45
   Phe Ala Pro Ala Thr Val Ala Asn Leu Gly Pro Gly Phe Asp Phe Leu
       50                  55                  60
   Gly Cys Ala Val Asp Gly Leu Gly Asp Ile Val Ser Val Lys Val Asp
   65                  70                  75                  80
   Pro Gln Val His Pro Gly Glu Ile Cys Ile Ser Asp Ile Ser Gly His
                   85                  90                  95
   Ala Pro Asn Lys Leu Ser Lys Asn Pro Leu Trp Asn Cys Ala Gly Ile
               100                 105                 110
   Ala Ala Ile Glu Val Met Lys Met Leu Ser Ile Arg Ser Val Gly Leu
           115                 120                 125
   Ser Leu Ser Leu Glu Lys Gly Leu Pro Leu Gly Ser Gly Leu Gly Ser
       130                 135                 140
   Ser Ala Ala Ser Ala Ala Ala Ala Ala Val Ala Val Asn Glu Leu Phe
   145                 150                 155                 160
   Gly Lys Lys Leu Ser Val Glu Glu Leu Val Leu Ala Ser Leu Lys Ser
                   165                 170                 175
   Glu Glu Lys Val Ser Gly Tyr His Ala Asp Asn Val Ala Pro Ser Ile
               180                 185                 190
   Met Gly Gly Phe Val Leu Ile Gly Ser Tyr Ser Pro Leu Glu Leu Met
           195                 200                 205
   Pro Leu Lys Phe Pro Ala Glu Lys Glu Leu Tyr Phe Val Leu Val Thr
       210                 215                 220
   Pro Glu Phe Glu Ala Pro Thr Lys Lys Met Arg Ala Ala Leu Pro Thr
   225                 230                 235                 240
   Glu Ile Gly Met Pro His His Val Trp Asn Cys Ser Gln Ala Gly Ala
                   245                 250                 255
   Leu Val Ala Ser Val Leu Gln Gly Asp Val Val Gly Leu Gly Lys Ala
               260                 265                 270
   Leu Ser Ser Asp Lys Ile Val Glu Pro Arg Arg Ala Pro Leu Ile Pro
           275                 280                 285
   Gly Met Glu Ala Val Lys Arg Ala Ala Ile Gln Ala Gly Ala Phe Gly
       290                 295                 300
   Cys Thr Ile Ser Gly Ala Gly Pro Thr Ala Val Ala Val Ile Asp Asp
   305                 310                 315                 320
   Glu Gln Thr Gly His Leu Ile Ala Lys His Met Ile Asp Ala Phe Leu
                   325                 330                 335
   His Val Gly Asn Leu Lys Ala Ser Ala Asn Val Lys Gln Leu Asp Arg
               340                 345                 350
   Leu Gly Ala Arg Arg Ile Pro Asn Thr Phe Ser Ser Leu Ser Leu Glu
           355                 360                 365
   Ala Cys Arg Phe Gln Glu Pro Asp Phe Phe Gln Leu Ala Arg Asn Thr
       370                 375                 380
   Leu Ser Ala Asp Arg Ser His Val Phe Glu Ile Ser Asp Gln Ser Ser
   385                 390                 395                 400
   Ile Leu Val Trp Arg Ser Glu Gln Glu Lys His Thr Gln Ala Gly Ser
                   405                 410                 415
   Ser Val Trp Val Val Glu Ile Ile Asp Glu Leu Lys Thr Ile Arg Ser
               420                 425                 430
   Val Leu Trp Thr Leu Lys His Val Leu Asp Phe Leu Cys Phe Val Phe
           435                 440                 445
   Ile Ile Phe Leu Ser Cys Tyr Leu Ser Gln Ser Ser Lys Arg Ser His
       450                 455                 460
   Phe Tyr Phe Leu Val Ser Leu Cys Leu Ile Leu Ala Phe Glu His Val
   465                 470                 475                 480
   Leu Phe Leu
(2) INFORMATION FOR SEQ ID NO:27:
   (i) SEQUENCE CHARACTERISTICS:
      (A) LENGTH: 438 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: wlln.pk0065.f2
   (xi) SEQUENCE DESCRIPTION: SEQ ID NO:27:
   CTCGAGTCGG AGAAGGCCGT CAGCGGCTTC CACGCCGACA ACATCGCCCC CGCCATCCTC    60
   GGCGGCTTCG TCCTCGTCCG CAGCTACGAC CCCTTTCACC TCGTCCCGCT TTCCTTCCCG   120
   CCAGCGCTCC GCCTCCACTT CGTCCTGGTC ACCCCCGACT TCGAGGCGCC CACGAGCAAG   180
   ATGCGCGCCG CGCTGCCCAG GCAGGTCGAC GTCCAGCAGC ACGTGCGCAA CTCCAGCCAG   240
   GCAGCGGCGC TCCGTGGCGG CGGTGCTGCA NGGGGACGCC GGGCTCATCG GTCCGCGATT   300
   TCTCCGACGG GCATCGTGGA CCCACCAAGG AACCCTCATA CCTGGCATGG CGGCCGTAAA   360
   GGCGGCGGCC TGCAACTGGA CGCTGGGTGC ACATTAACGG GCGGGCCCAC ATGGTGGCTC   420
   NCAGNGAAGA GAGGGGAG                                                 438
(2) INFORMATION FOR SEQ ID NO:28:
   (i) SEQUENCE CHARACTERISTICS:
      (A) LENGTH: 84 amino acids
      (B) TYPE: amino acid
      (C) STRANDEDNESS:
      (D) TOPOLOGY: linear
   (ii) MOLECULE TYPE: peptide
   (vii) IMMEDIATE SOURCE:
      (B) CLONE: wlln.pk0065.f2
   (xi) SEQUENCE DESCRIPTION: SEQ ID NO:28:
   Leu Glu Ser Glu Lys Ala Val Ser Gly Phe His Ala Asp Asn Ile Ala
   1               5                   10                  15
   Pro Ala Ile Leu Gly Gly Phe Val Leu Val Arg Ser Tyr Asp Pro Phe
               20                  25                  30
   His Leu Val Pro Leu Ser Phe Pro Pro Ala Leu Arg Leu His Phe Val
           35                  40                  45
   Leu Val Thr Pro Asp Phe Glu Ala Pro Thr Ser Lys Met Arg Ala Ala
       50                  55                  60
   Leu Pro Arg Gln Val Asp Val Gln Gln His Val Arg Asn Ser Ser Gln
   65                  70                  75                  80
   Ala Ala Ala Leu
(2) INFORMATION FOR SEQ ID NO:29:
   (i) SEQUENCE CHARACTERISTICS:
      (A) LENGTH: 300 amino acids
      (B) TYPE: amino acid
      (C) STRANDEDNESS: not relevant
      (D) TOPOLOGY: linear
   (ii) MOLECULE TYPE: peptide
   (vi) ORIGINAL SOURCE:
      (A) ORGANISM: Methanococcus jannashii
   (xi) SEQUENCE DESCRIPTION: SEQ ID NO:29:
   Met Arg Glu Ile Met Lys Val Arg Val Lys Ala Pro Cys Thr Ser Ala
   1               5                   10                  15
   Asn Leu Gly Val Gly Phe Asp Val Phe Gly Leu Cys Leu Lys Glu Pro
               20                  25                  30
   Tyr Asp Val Ile Glu Val Glu Ala Ile Asp Asp Lys Glu Ile Ile Ile
           35                  40                  45
   Glu Val Asp Asp Lys Asn Ile Pro Thr Asp Pro Asp Lys Asn Val Ala
       50                  55                  60
   Gly Ile Val Ala Lys Lys Met Ile Asp Asp Phe Asn Ile Gly Lys Gly
   65                  70                  75                  80
   Val Lys Ile Thr Ile Lys Lys Gly Val Lys Ala Gly Ser Gly Leu Gly
                   85                  90                  95
   Ser Ser Ala Ala Ser Ser Ala Gly Thr Ala Tyr Ala Ile Asn Glu Leu
               100                 105                 110
   Phe Lys Leu Asn Leu Asp Lys Leu Lys Leu Val Asp Tyr Ala Ser Tyr
           115                 120                 125
   Gly Glu Leu Ala Ser Ser Gly Ala Lys His Ala Asp Asn Val Ala Pro
       130                 135                 140
   Ala Ile Phe Gly Gly Phe Thr Met Val Thr Asn Tyr Glu Pro Leu Glu
   145                 150                 155                 160
   Val Leu His Ile Pro Ile Asp Phe Lys Leu Asp Ile Leu Ile Ala Ile
                   165                 170                 175
   Pro Asn Ile Ser Ile Asn Thr Lys Glu Ala Arg Glu Ile Leu Pro Lys
               180                 185                 190
   Ala Val Gly Leu Lys Asp Leu Val Asn Asn Val Gly Lys Ala Cys Gly
           195                 200                 205
   Met Val Tyr Ala Leu Tyr Asn Lys Asp Lys Ser Leu Phe Gly Arg Tyr
       210                 215                 220
   Met Met Ser Asp Lys Val Ile Glu Pro Val Arg Gly Lys Leu Ile Pro
   225                 230                 235                 240
   Asn Tyr Phe Lys Ile Lys Glu Glu Val Lys Asp Lys Val Tyr Gly Ile
                   245                 250                 255
   Thr Ile Ser Gly Ser Gly Pro Ser Ile Ile Ala Phe Pro Lys Glu Glu
               260                 265                 270
   Phe Ile Asp Glu Val Glu Asn Ile Leu Arg Asp Tyr Tyr Glu Asn Thr
           275                 280                 285
   Ile Arg Thr Glu Val Gly Lys Gly Val Glu Val Val
       290                 295                 300
(2) INFORMATION FOR SEQ ID NO:30:
   (i) SEQUENCE CHARACTERISTICS:
      (A) LENGTH: 1362 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: se3.05h06
   (xi) SEQUENCE DESCRIPTION: SEQ ID NO:30:
   ACTTTGTAGT TCGTAGATAG CCGATGTGCT TGTCTTAGTG TGTCAGTCAT TCCTGTTCCT     60
   CAAGTCAAGC TTTGTAGTGA GCAGATATAA TGGCTGTTGA AAGGTCCGGA ATTGCCAAAG    120
   ATGTTACGGA ATTGATTGGT AAAACCCCAT TAGTATATCT AAATAAACTT GCGGATGGTT    180
   GTGTTGCCCG GGTTGCTGCT AAACTGGAGT TGATGGAGCC ATGCTCTAGT GTGAAGGACA    240
   GGATTGGGTA TAGTATGATT GCTGATGCAG AAGAGAAGGG ACTTATCACA CCTGGAAAGA    300
   GTGTCCTCAT TGAGCCAACA AGTGGTAATA CTGGCATTGG ATTAGCCTTC ATGGCAGCAG    360
   CCAGGGGTTA CAAGCTCATA ATTACAATGC CTGCTTCTAT GAGTCTTGAG AGAAGAATCA    420
   TTCTATTAGC TTTTGGAGCT GAGTTGGTTC TGACAGATCC TGCTAAGGGA ATGAAAGGTG    480
   CTGTTCAGAA GGCTGAAGAG ATATTGGCTA AGACGCCCAA TGCCTACATA CTTCAACAAT    540
   TTGAAAACCC TGCCAATCCC AAGGTTCATT ATGAAACCAC TGGTCCAGAG ATATGGAAAG    600
   GCTCCGATGG GAAAATTGAT GCATTTGTTT CTGGGATAGG CACTGGTGGT ACAATAACAG    660
   GTGCTGGAAA ATATCTTAAA GAGCAGAATC CGAATATAAA GCTGATTGGT GTGGAACCAG    720
   TTGAAAGTCC AGTGCTCTCA GGAGGAAAGC CTGGTCCACA CAAGATTCAA GGGATTGGTG    780
   CTGGTTTTAT CCCTGGTGTC TTGGAAGTCA ATCTTCTTGA TGAAGTTGTT CAAATATCAA    840
   GTGATGAAGC AATAGAAACT GCAAAGCTTC TTGCGCTTAA AGAAGGCCTA TTTGTGGGAA    900
   TATCTTCCGG AGCTGCAGCT GCTGCTGCTT TTCAGATTGC AAAAAGACCA GAAAATGCCG    960
   GGAAGCTTAT TGTTGCCGTT TTTCCCAGCT TCGGGGAGAG GTACCTGTCC TCCGTGCTAT   1020
   TTGAGTCAGT GAGACGCGAA GCTGAAAGCA TGACTTTTGA GCCCTGAATT CCCGTTTAAG   1080
   GCTCTCACTA CTGAATTTTC TTGTTACTTG TACCAGGCTT TAACTAGATT GTTAGAGTAC   1140
   TACTGTTTGT GACTCTGACT CTAAAATAAA ACTTGCTCCA AAAGACTAGT TTTTCTTGAT   1200
   GCCCCTGGAG CGATAATTTT GTGCCTGCAA CATTAAAAAG TATTCAAAGT TGCTTATAAG   1260
   TAACATGTTT CATCTTTTGT TGTTGTTGAG ACGAACACGG ATGAGGTCAT AATACTATGT   1320
   TTCTGATTTC CTTTGGTAGG GAAAAAAAAA AAAAAAAAAA AA                      1362
(2) INFORMATION FOR SEQ ID NO:31:
   (i) SEQUENCE CHARACTERISTICS:
      (A) LENGTH: 325 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: se3.05h06
   (xi) SEQUENCE DESCRIPTION: SEQ ID NO:31:
   Met Ala Val Glu Arg Ser Gly Ile Ala Lys Asp Val Thr Glu Leu Ile
   1               5                   10                  15
   Gly Lys Thr Pro Leu Val Tyr Leu Asn Lys Leu Ala Asp Gly Cys Val
               20                  25                  30
   Ala Arg Val Ala Ala Lys Leu Glu Leu Met Glu Pro Cys Ser Ser Val
           35                  40                  45
   Lys Asp Arg Ile Gly Tyr Ser Met Ile Ala Asp Ala Glu Glu Lys Gly
       50                  55                  60
   Leu Ile Thr Pro Gly Lys Ser Val Leu Ile Glu Pro Thr Ser Gly Asn
   65                  70                  75                  80
   Thr Gly Ile Gly Leu Ala Phe Met Ala Ala Ala Arg Gly Tyr Lys Leu
                   85                  90                  95
   Ile Ile Thr Met Pro Ala Ser Met Ser Leu Glu Arg Arg Ile Ile Leu
               100                 105                 110
   Leu Ala Phe Gly Ala Glu Leu Val Leu Thr Asp Pro Ala Lys Gly Met
           115                 120                 125
   Lys Gly Ala Val Gln Lys Ala Glu Glu Ile Leu Ala Lys Thr Pro Asn
       130                 135                 140
   Ala Tyr Ile Leu Gln Gln Phe Glu Asn Pro Ala Asn Pro Lys Val His
   145                 150                 155                 160
   Tyr Glu Thr Thr Gly Pro Glu Ile Trp Lys Gly Ser Asp Gly Lys Ile
                   165                 170                 175
   Asp Ala Phe Val Ser Gly Ile Gly Thr Gly Gly Thr Ile Thr Gly Ala
               180                 185                 190
   Gly Lys Tyr Leu Lys Glu Gln Asn Pro Asn Ile Lys Leu Ile Gly Val
           195                 200                 205
   Glu Pro Val Glu Ser Pro Val Leu Ser Gly Gly Lys Pro Gly Pro His
       210                 215                 220
   Lys Ile Gln Gly Ile Gly Ala Gly Phe Ile Pro Gly Val Leu Glu Val
   225                 230                 235                 240
   Asn Leu Leu Asp Glu Val Val Gln Ile Ser Ser Asp Glu Ala Ile Glu
                   245                 250                 255
   Thr Ala Lys Leu Leu Ala Leu Lys Glu Gly Leu Phe Val Gly Ile Ser
               260                 265                 270
   Ser Gly Ala Ala Ala Ala Ala Ala Phe Gln Ile Ala Lys Arg Pro Glu
           275                 280                 285
   Asn Ala Gly Lys Leu Ile Val Ala Val Phe Pro Ser Phe Gly Glu Arg
       290                 295                 300
   Tyr Leu Ser Ser Val Leu Phe Glu Ser Val Arg Arg Glu Ala Glu Ser
   305                 310                 315                 320
   Met Thr Phe Glu Pro
                   325
(2) INFORMATION FOR SEQ ID NO:32:
   (i) SEQUENCE CHARACTERISTICS:
      (A) LENGTH: 325 amino acids
      (B) TYPE: amino acid
      (C) STRANDEDNESS: not relevant
      (D) TOPOLOGY: linear
   (ii) MOLECULE TYPE: peptide
   (vi) ORIGINAL SOURCE:
      (A) ORGANISM: Citrullus lanatus
   (xi) SEQUENCE DESCRIPTION: SEQ ID NO:32:
   Met Ala Asp Ala Lys Ser Thr Ile Ala Lys Asp Val Thr Glu Leu Ile
   1               5                   10                  15
   Gly Asn Thr Pro Leu Val Tyr Leu Asn Arg Val Val Asp Gly Cys Val
               20                  25                  30
   Ala Arg Val Ala Ala Lys Leu Glu Met Met Glu Pro Cys Ser Ser Val
           35                  40                  45
   Lys Asp Arg Ile Gly Tyr Ser Met Ile Ser Asp Ala Glu Asn Lys Gly
       50                  55                  60
   Leu Ile Thr Pro Gly Glu Ser Val Leu Ile Glu Pro Thr Ser Gly Asn
   65                  70                  75                  80
   Thr Gly Ile Gly Leu Ala Phe Ile Ala Ala Ala Lys Gly Tyr Arg Leu
                   85                  90                  95
   Ile Ile Cys Met Pro Ala Ser Met Ser Leu Glu Arg Arg Thr Ile Leu
               100                 105                 110
   Arg Ala Phe Gly Ala Glu Leu Val Leu Thr Asp Pro Ala Arg Gly Met
           115                 120                 125
   Lys Gly Ala Val Gln Lys Ala Glu Glu Ile Lys Ala Lys Thr Pro Asn
       130                 135                 140
   Ser Tyr Ile Leu Gln Gln Phe Glu Asn Pro Ala Asn Pro Lys Ile His
   145                 150                 155                 160
   Tyr Glu Thr Thr Gly Pro Glu Ile Trp Arg Gly Ser Gly Gly Lys Ile
                   165                 170                 175
   Asp Ala Leu Val Ser Gly Ile Gly Thr Gly Gly Thr Val Thr Gly Ala
               180                 185                 190
   Gly Lys Tyr Leu Lys Glu Gln Asn Pro Asn Ile Lys Leu Tyr Gly Val
           195                 200                 205
   Glu Pro Val Glu Ser Ala Ile Leu Ser Gly Gly Lys Pro Gly Pro His
       210                 215                 220
   Lys Ile Gln Gly Ile Gly Ala Gly Phe Ile Pro Gly Val Leu Asp Val
   225                 230                 235                 240
   Asn Leu Leu Asp Glu Val Ile Gln Val Ser Ser Glu Glu Ser Ile Glu
                   245                 250                 255
   Thr Ala Lys Leu Leu Ala Leu Lys Glu Gly Leu Leu Val Gly Ile Ser
               260                 265                 270
   Ser Gly Ala Ala Ala Ala Ala Ala Ile Arg Ile Ala Lys Arg Pro Glu
           275                 280                 285
   Asn Ala Gly Lys Leu Ile Val Ala Val Phe Pro Ser Phe Gly Glu Arg
       290                 295                 300
   Tyr Leu Ser Thr Val Leu Phe Glu Ser Val Lys Arg Glu Thr Glu Asn
   305                 310                 315                 320
   Met Val Phe Glu Pro
(2) INFORMATION FOR SEQ ID NO:33:
   (i) SEQUENCE CHARACTERISTICS:
      (A) LENGTH: 547 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: cenl.pk0061.d4
   (xi) SEQUENCE DESCRIPTION: SEQ ID NO:33:
   GCCTTATGGC TAAGCTTGAG AAGGCGGATC AGGCATTCTG CTTCACCAGT GGGATGGCAG    60
   CACTAGCTGC AGTAACACAC CTCCTTAAGT CTGGACAAGA AATAGTTGCT GGAGAGGACA   120
   TATATGGTGG CTCAGACCGT CTGCTCTCAC AAGTTGCCCC GAGACATGGG ATTGTAGTAA   180
   AACGAATTGA TACAACCAAA ATTAGTGAGG TAACTTCTGC AATTGGGGCC TTGGACTAAA   240
   CTAAGTATGG CTTTGAAAAN CCCACCATCC CCGTCCTACA AATTACTGGA TATAAAGAAA   300
   ATAGCNAGAG ATAGTCATTA CAATGGGGCT CCTTGTTTTA AGTAGACAAC AGCACATGTC   360
   TCCCTGTGCT CTCCCNGTCC TCNTAAAACT TTGGGCCAAA TATNGGTTTG CACCCCAAGC   420
   AACCAATTTA TNCTGGGCAT AGCGTNCTTA TGGCNNGGAT CCTTGCCGGG AAGGGGTGAA   480
   AGCACTTGGC TAAAGAGATG CATTCCTCNA AAANCTGAAG GNTAAGTTTG GACATTNGAT   540
   GCCGGTT                                                             547
(2) INFORMATION FOR SEQ ID NO:34:
   (i) SEQUENCE CHARACTERISTICS:
      (A) LENGTH: 223 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: cenl.pk0061.d9
   (xi) SEQUENCE DESCRIPTION: SEQ ID NO:34:
   Ile Ala His Ser His Gly Ala Leu Val Leu Val Asp Asn Ser Ile Met
   1               5                   10                  15
   Ser Pro Val Leu Ser Arg Pro Ile Glu Leu Gly Ala Asp Ile Val Met
               20                  25                  30
   His Ser Ala Thr Lys Phe Ile Ala Gly His Ser Asp Leu Met Ala Gly
           35                  40                  45
   Ile Leu Ala Val Lys Gly Glu Ser Leu Ala Lys Glu Val Gly Phe Leu
       50                  55                  60
   Gln Asn Ala Glu Gly Ser Gly Leu Ala Pro Phe Asp Cys Trp Leu Cys
   65                  70                  75                  80
   Leu Arg Gly Ile Lys Thr Met Ala Leu Arg Val Glu Lys Gln Gln Ala
                   85                  90                  95
   Asn Ala Gln Lys Ile Ala Glu Phe Leu Ala Ser His Pro Arg Val Lys
               100                 105                 110
   Gln Val Asn Tyr Ala Gly Leu Pro Asp His Pro Gly Arg Ala Leu His
           115                 120                 125
   Tyr Ser Gln Ala Lys Gly Ala Gly Ser Val Leu Ser Phe Leu Thr Gly
       130                 135                 140
   Ser Leu Ala Leu Ser Lys His Val Val Glu Thr Thr Lys Tyr Phe Ser
   145                 150                 155                 160
   Val Thr Val Ser Phe Gly Ser Val Lys Ser Leu Ile Ser Leu Pro Cys
                   165                 170                 175
   Phe Met Ser His Ala Ser Ile Pro Ala Ser Val Arg Glu Glu Arg Gly
               180                 185                 190
   Leu Thr Asp Asp Leu Val Arg Ile Ser Val Gly Ile Glu Asp Val Glu
           195                 200                 205
   Asp Leu Ile Ala Asp Leu Asp Arg Ala Leu Arg Thr Gly Pro Val
       210                 215                 220
(2) INFORMATION FOR SEQ ID NO:35:
   (i) SEQUENCE CHARACTERISTICS:
      (A) LENGTH: 547 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: rlr12.pk0026.gl
   (xi) SEQUENCE DESCRIPTION: SEQ ID NO:35:
   GCCTTATGGC TAAGCTTGAG AAGGCGGATC AGGCATTCTG CTTCACCAGT GGGATGGCAG     60
   CACTAGCTGC AGTAACACAC CTCCTTAAGT CTGGACAAGA AATAGTTGCT GGAGAGGACA    120
   TATATGGTGG CTCAGACCGT CTGCTCTCAC AAGTTGCCCC GAGACATGGG ATTGTAGTAA    180
   AACGAATTGA TACAACCAAA ATTAGTGAGG TAACTTCTGC AATTGGGGCC TTGGACTAAA    240
   CTAAGTATGG CTTTGAAAAN CCCACCATCC CCGTCCTACA AATTACTGGA TATAAAGAAA    300
   ATAGCNAGAG ATAGTCATTA CAATGGGGCT CCTTGTTTTA AGTAGACAAC AGCACATGTC    360
   TCCCTGTGCT CTCCCNGTCC TCNTAAAACT TTGGGCCAAA TATNGGTTTG CACCCCAAGC    420
   AACCAATTTA TNCTGGGCAT AGCGTNCTTA TGGCNNGGAT CCTTGCCGGG AAGGGGTGAA    480
   AGCACTTGGC TAAAGAGATG CATTCCTCNA AAANCTGAAG GNTAAGTTTG GACATTNGAT    540
   GCCGGTT                                                              547
(2) INFORMATION FOR SEQ ID NO:36:
   (i) SEQUENCE CHARACTERISTICS:
      (A) LENGTH: 75 amino acids
      (B) TYPE: amino acid
      (C) STRANDEDNESS: not relevant
      (D) TOPOLOGY: linear
   (ii) MOLECULE TYPE: peptide
   (vii) IMMEDIATE SOURCE:
      (B) CLONE: rlr12.pk0026.gl
   (xi) SEQUENCE DESCRIPTION: SEQ ID NO:36:
   Leu Met Ala Lys Leu Glu Lys Ala Asp Gln Ala Phe Cys Phe Thr Ser
   1               5                   10                  15
   Gly Met Ala Ala Leu Ala Ala Val Thr His Leu Leu Lys Ser Gly Gln
               20                  25                  30
   Glu Ile Val Ala Gly Glu Asp Ile Tyr Gly Gly Ser Asp Arg Leu Leu
           35                  40                  45
   Ser Gln Val Ala Pro Arg His Gly Ile Val Val Lys Arg Ile Asp Thr
       50                  55                  60
   Thr Lys Ile Ser Glu Val Thr Ser Ala Ile Gly
   65                  70                  75
(2) INFORMATION: FOR SEQ ID NO:37:
   (i) SEQUENCE CHARACTERISTICS:
      (A) LENGTH: 1733 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: sfl1.pk0012.c4
   (xi) SEQUENCE DESCRIPTION: SEQ ID NO:37:
(2) INFORMATION FOR SEQ ID NO:38:
   (i) SEQUENCE CHARACTERISTICS:
      (A) LENGTH: 467 amino acids
      (B) TYPE: amino acid
      (C) STRANDEDNESS: not relevant
      (D) TOPOLOGY: linear
   (ii. MOLECULE TYPE: peptide
   (vii. IMMEDIATE SOURCE:
      (B) CLONE: sfll.pk0012.c4
   (xi) SEQUENCE DESCRIPTION: SEQ ID NO:38:
   Met Phe Ser Ser Ala Ile Ser Gln Lys Pro Phe Leu Gln Ser Leu Val
   1               5                   10                  15
   Ile Asp Arg Tyr Ala Gln Ser Thr Thr Ala Ala Thr Arg Trp Glu Cys
               20                  25                  30
   Leu Gly Phe Asn Lys Ser Glu Asn Phe Ser Thr Lys Arg Val Leu Arg
           35                  40                  45
   Ala Glu Gly Phe Lys Leu Asn Cys Leu Val Glu Asn Arg Glu Met Glu
       50                  55                  60
   Val Glu Ser Ser Ser Ser Ser Leu Val Asp Asp Ala Ala Met Ser Leu
   65                  70                  75                  80
   Ser Glu Glu Asp Leu Gly Glu Pro Ser Ile Ser Thr Met Val Met Asn
                   85                  90                  95
   Phe Glu Ser Lys Phe Asp Pro Phe Gly Ala Ile Ser Thr Pro Leu Tyr
               100                 105                 110
   Gln Thr Ala Thr Phe Lys Gln Pro Ser Ala Ile Glu Asn Gly Pro Tyr
           115                 120                 125
   Asp Tyr Thr Arg Ser Gly Asn Pro Thr Arg Asp Ala Leu Glu Ser Leu
       130                 135                 140
   Leu Ala Lys Leu Asp Lys Ala Asp Arg Ala Leu Cys Phe Thr Ser Gly
   145                 150                 155                 160
   Met Ala Ala Leu Ser Ala Val Val Arg Leu Val Gly Thr Gly Glu Glu
                   165                 170                 175
   Ile Val Thr Gly Asp Asp Val Tyr Gly Gly Ser Asp Arg Leu Leu Ser
               180                 185                 190
   Gln Val Val Pro Arg Thr Gly Ile Val Val Lys Arg Val Asn Thr Cys
           195                 200                 205
   Asp Leu Asp Glu Val Ala Ala Ala Ile Gly Leu Arg Thr Lys Leu Val
       210                 215                 220
   Trp Leu Glu Ser Pro Thr Asn Pro Arg Leu Gln Ile Ser Asp Ile Arg
   225                 230                 235                 240
   Lys Ile Ser Glu Met Ala His Ser His Gly Ala Leu Val Leu Val Asp
                   245                 250                 255
   Asn Ser Ile Met Ser Pro Val Leu Ser Gln Pro Leu Glu Leu Gly Ala
               260                 265                 270
   Asp Ile Val Met His Ser Ala Thr Lys Phe Ile Ala Gly His Ser Asp
           275                 280                 285
   Ile Met Ala Gly Val Leu Ala Val Lys Gly Glu Lys Leu Gly Lys Glu
       290                 295                 300
   Met Tyr Phe Leu Gln Asn Ala Glu Gly Ser Gly Leu Ala Pro Phe Asp
   305                 310                 315                 320
   Cys Trp Leu Cys Leu Arg Gly Ile Lys Thr Met Ala Leu Arg Ile Glu
                   325                 330                 335
   Lys Gln Gln Asp Asn Ala Gln Lys Ile Ala Glu Phe Leu Ala Ser His
               340                 345                 350
   Pro Arg Val Lys Glu Val Asn Tyr Ala Gly Leu Pro Gly His Pro Gly
           355                 360                 365
   Arg Asp Leu His Tyr Ser Gln Ala Lys Gly Ala Gly Ser Val Leu Ser
       370                 375                 380
   Phe Leu Thr Gly Ser Leu Ala Leu Ser Lys His Ile Val Glu Thr Thr
   385                 390                 395                 400
   Lys Tyr Phe Ser Ile Thr Val Ser Phe Gly Ser Val Lys Ser Leu Ile
                   405                 410                 415
   Ser Met Pro Cys Phe Met Ser His Ala Ser Ile Pro Ala Ala Val Arg
               420                 425                 430
   Glu Ala Arg Gly Leu Thr Glu Asp Leu Val Arg Ile Ser Val Gly Ile
           435                 440                 445
   Glu Asp Val Asn Asp Leu Ile Ala Asp Leu Gly Asn Ala Leu Arg Thr
       450                 455                 460
   Gly Pro Leu
   465
(2) INFORMATION FOR SEQ ID NO:39:
   (i) SEQUENCE CHARACTERISTICS:
      (A) LENGTH: 637 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: wrl.pk0091.g6
   (xi) SEQUENCE DESCRIPTION: SEQ ID NO:39:
   AGCGTGGCCA CGATACTGAC CAGCTTCGAG AACTCGTTCG ACAAGTATGG GGCTCTCAGC    60
   ACGCCGCTGT ACCAGACGGC CACCTTCAAG CAGCCTTCAG CAACCGTTAA TGGAGCTTAT   120
   GATTATACTA GAAGTGGCAA CCCTACTCGT GATGTTCTCC AGAGCCTTAT GGCTAAGCTC   180
   GAGAAGGCAG ACCAAGCATT CTGCTTCACT AGTGGGATGG CATCACTGGG CTGCAGTAAC   240
   ACACCTCCTT CAGGCTGGAC AAGAAATAGT TGCTGGAGAG GACATATATG GTGGTCTGAT   300
   CGTCTGCTCT CACAAGTTGT CCCAAGAAAT GGAATTGTAG TAAAACGGGT CGATACAACT   360
   AAAATTAACG ACGTGACTGC TGCATCGGAC CCTTGACTAN ACTAGTTTGG TTGAAANCCA   420
   CAATCCTCGT CAACAATTAC TGTATAAGAA ATCTCAGGGA TACTCATCCA TGGGGACTGG   480
   TTTGGNGGCA ANNTTCATGT CCCANGGCTA CCTGGCCNAT AAANTGGGGN ANTATGGGAG   540
   CATCAGTACA AATTATNCTG GCNATGTCTA GGTGGATCTC NTAAGGGGAA NTTGGNAGGA   600
   TTCTTCAAAA CCTAGTNGGT TGACTTATGT GGTTGTT                            637
(2) INFORMATION FOR SEQ ID NO:40:
   (i) SEQUENCE CHARACTERISTICS:
      (A) LENGTH: 131 amino acids
      (B) TYPE: amino acid
      (C) STRANDEDNESS not relevant
      (D) TOPOLOGY: linear
   (ii) MOLECULE TYPE: peptide
   (vii) IMMEDIATE SOURCE:
      (B) CLONE: wrl.pk0091.g6
   (xi) SEQUENCE DESCRIPTION: SEQ ID NO:40:
   Ser Val Ala Thr Ile Leu Thr Ser Phe Glu Asn Ser Phe Asp Lys Tyr
   1               5                   10                  15
   Gly Ala Leu Ser Thr Pro Leu Tyr Gln Thr Ala Thr Phe Lys Gln Pro
               20                  25                  30
   Ser Ala Thr Val Asn Gly Ala Tyr Asp Tyr Thr Arg Ser Gly Asn Pro
           35                  40                  45
   Thr Arg Asp Val Leu Gln Ser Leu Met Ala Lys Leu Glu Lys Ala Asp
       50                  55                  60
   Gln Ala Phe Cys Phe Thr Ser Gly Met Ala Ser Leu Xaa Ala Val Thr
   65                  70                  75                  80
   His Leu Leu Gln Ala Gly Gln Glu Ile Val Ala Gly Glu Asp Ile Tyr
                   85                  90                  95
   Gly Gly Xaa Asp Arg Leu Leu Ser Gln Val Val Pro Arg Asn Gly Ile
               100                 105                 110
   Val Val Lys Arg Val Asp Thr Thr Lys Ile Asn Asp Val Thr Ala Ala
           115                 120                 125
   Ser Asp Pro
       130
(2) INFORMATION FOR SEQ ID NO:41:
   (i) SEQUENCE CHARACTERISTICS:
      (A) LENGTH: 464 amino acids
      (B) TYPE: amino acid
      (C) STKANOEDNESS: single
      (D) TOPOLOGY: linear
   (ii) MOLECULE TYPE: cDNA
   (vi) ORIGINAL SOURCE:
      (A) ORGANISM: Arabidopsis thaliana
   (xi) SEQUENCE DESCRIPTION: SEQ ID NO:41:
   Met Thr Ser Ser Leu Ser Leu His Ser Ser Phe Val Pro Ser Phe Ala
   1               5                   10                  15
   Asp Leu Ser Asp Arg Gly Leu Ile Ser Lys Asn Ser Pro Thr Ser Val
               20                  25                  30
   Ser Ile Ser Lys Val Pro Thr Trp Glu Lys Lys Gln Ile Ser Asn Arg
           35                  40                  45
   Asn Ser Phe Lys Leu Asn Cys Val Met Glu Lys Ser Val Asp Gly Gln
       50                  55                  60
   Thr His Ser Thr Val Asn Asn Thr Thr Asp Ser Leu Asn Thr Met Asn
   65                  70                  75                  80
   Ile Lys Glu Glu Ala Ser Val Ser Thr Leu Leu Val Asn Leu Asp Asn
                   85                  90                  95
   Lys Phe Asp Pro Phe Asp Ala Met Ser Thr Pro Leu Tyr Gln Thr Ala
               100                 105                 110
   Thr Phe Lys Gln Pro Ser Ala Ile Glu Asn Gly Pro Tyr Asp Tyr Thr
           115                 120                 125
   Arg Ser Gly Asn Pro Thr Arg Asp Ala Leu Glu Ser Leu Leu Ala Lys
       130                 135                 140
   Leu Asp Lys Ala Asp Arg Ala Phe Cys Phe Thr Ser Gly Met Ala Ala
   145                 150                 155                 160
   Leu Ser Ala Val Thr His Leu Ile Lys Asn Gly Glu Glu Ile Val Ala
                   165                 170                 175
   Gly Asp Asp Val Tyr Gly Gly Ser Asp Arg Leu Leu Ser Gln Val Val
               180                 185                 190
   Pro Arg Ser Gly Val Val Val Lys Arg Val Asn Thr Thr Lys Leu Asp
           195                 200                 205
   Glu Val Ala Ala Ala Ile Gly Pro Gln Thr Lys Leu Val Trp Leu Glu
       210                 215                 220
   Ser Pro Thr Asn Pro Arg Gln Gln Ile Ser Asp Ile Arg Lys Ile Ser
   225                 230                 235                 240
   Glu Met Ala His Ala Gln Gly Ala Leu Val Leu Val Asp Asn Ser Ile
                   245                 250                 255
   Met Ser Pro Val Leu Ser Arg Pro Leu Glu Leu Gly Ala Asp Ile Val
               260                 265                 270
   Met His Ser Ala Thr Lys Phe Ile Ala Gly His Ser Asp Val Met Ala
           275                 280                 285
   Gly Val Leu Ala Val Lys Gly Glu Lys Leu Ala Lys Glu Val Tyr Phe
       290                 295                 300
   Leu Gln Asn Ser Glu Gly Ser Gly Leu Ala Pro Phe Asp Cys Trp Leu
   305                 310                 315                 320
   Cys Leu Arg Gly Ile Lys Thr Met Ala Leu Arg Ile Glu Lys Gln Gln
                   325                 330                 335
   Glu Asn Ala Arg Lys Ile Ala Met Tyr Leu Ser Ser His Pro Arg Val
               340                 345                 350
   Lys Lys Val Tyr Tyr Ala Gly Leu Pro Asp His Pro Gly His His Leu
           355                 360                 365
   His Phe Ser Gln Ala Lys Gly Ala Gly Ser Val Phe Ser Phe Ile Thr
       370                 375                 380
   Gly Ser Val Ala Leu Ser Lys His Leu Val Glu Thr Thr Lys Tyr Phe
   385                 390                 395                 400
   Ser Ile Ala Val Ser Phe Gly Ser Val Lys Ser Leu Ile Ser Met Pro
                   405                 410                 415
   Cys Phe Met Ser His Ala Ser Ile Pro Ala Glu Val Arg Glu Ala Arg
               420                 425                 430
   Gly Leu Thr Glu Asp Leu Val Arg Ile Ser Ala Gly Ile Glu Asp Val
           435                 440                 445
   Asp Asp Leu Ile Ser Asp Leu Asp Ile Ala Phe Lys Thr Phe Pro Leu
       450                 455                 460